(19) **Europäisches Patentamt**
**European Patent Office**
**Office européen des brevets**

(11) **EP 2 518 084 B1**

(12) **EUROPEAN PATENT SPECIFICATION**

(45) Date of publication and mention
of the grant of the patent:
**30.11.2016 Bulletin 2016/48**

(21) Application number: **10839612.8**

(22) Date of filing: **27.12.2010**

(51) Int Cl.:
**G01N 33/74** *(2006.01)*    **C07K 16/26** *(2006.01)*

(86) International application number:
**PCT/JP2010/073633**

(87) International publication number:
**WO 2011/078384 (30.06.2011 Gazette 2011/26)**

(54) **METHOD FOR MEASURING HUMAN INSULIN AND MEASUREMENT REAGENT**

VERFAHREN ZUR MESSUNG DES MENSCHLICHEN INSULINS UND MESSREAGENS DAFÜR

PROCÉDÉ POUR MESURER L'INSULINE HUMAINE ET RÉACTIF DE MESURE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.12.2009 JP 2009295260**

(43) Date of publication of application:
**31.10.2012 Bulletin 2012/44**

(83) **Declaration under Rule 32(1) EPC (expert
solution)**

(73) Proprietor: **Sekisui Medical Co., Ltd.
Tokyo 103-0027 (JP)**

(72) Inventors:
• **SHIMIZU, Tomo
Ryugasaki-shi
Ibaraki 301-0852 (JP)**
• **KONDOU, Junichi
Ryugasaki-shi
Ibaraki 301-0852 (JP)**
• **NAKAMURA, Yasushi
Ryugasaki-shi
Ibaraki 301-0852 (JP)**
• **YAMAMOTO, Mitsuaki
Ryugasaki-shi
Ibaraki 301-0852 (JP)**

(74) Representative: **Hoffmann Eitle
Patent- und Rechtsanwälte PartmbB
Arabellastraße 30
81925 München (DE)**

(56) References cited:

• **ALLAUZEN S ET AL: "Epitope mapping and
binding analysis of insulin-specific monoclonal
antibodies using a biosensor approach",
JOURNAL OF IMMUNOLOGICAL METHODS,
ELSEVIER SCIENCE PUBLISHERS
B.V.,AMSTERDAM, NL, vol. 183, no. 1, 14 June
1995 (1995-06-14) , pages 27-32, XP004021022,
ISSN: 0022-1759, DOI:
10.1016/0022-1759(95)00020-B**
• **OWEN WILLIAM E ET AL: "Cross-reactivity of
three recombinant insulin analogs with five
commercial insulin immunoassays", CLINICAL
CHEMISTRY, AMERICAN ASSOCIATION FOR
CLINICAL CHEMISTRY, WASHINGTON, DC, vol.
50, no. 1, 1 January 2004 (2004-01-01), pages
257-259, XP009169156, ISSN: 0009-9147**
• **SAPIN R: "Insulin assays: Previously known and
new analytical features", CLINICAL
LABORATORY 2003 DE, vol. 49, no. 3-4, 2003,
pages 113-121, XP9169162, ISSN: 1433-6510**
• **LINDSTRÖM, T. ET AL: "Use of a novel
double-antibody technique to describe the
pharmacokinetics of rapid-acting insulin
analogs.", DIABETES CARE, vol. 25, no. 6, June
2002 (2002-06), pages 1049-1054, XP9169183,**
• **MIRZA I.H. ET AL.: 'Antigenicity of the carboxyl
terminus of insulin: Isolation of human
insulin-specific monoclonal antibodies'
IMMUNOLOGY vol. 65, 1988, pages 43 - 46,
XP009169066**

**(Cont. next page)**

- TADASHI KOBAYASHI ET AL.: 'Recent progress in laboratory tests of endocrine function. Measurement and clinical significance of insulin antibody and anti-insulin receptor antibody' THE JAPANESE JOURNAL OF CLINICAL PATHOLOGY vol. 33, 1985, pages 513 - 518, XP009169065

- SASAKI, ATSUKO ET AL.: 'Monoclonal antibodies identify tertiary structure differences in insulin molecules' JIKEIKAI MED J vol. 34, 1987, pages 337 - 357, XP009169061

**Description**

TECHNICAL FIELD

The present invention relates to an antibody specifically reactive with human insulin. The present invention also relates to a human insulin assay and an assay reagent using the monoclonal antibody specifically reactive with human insulin.

BACKGROUND ART

Insulin is a peptide hormone (molecular weight: approximately 5800) that is produced via a precursor, proinsulin, in the beta cells in the pancreatic islets of Langerhans, and is made up of an α-chain (also referred to as A-chain), i.e., a peptide consisting of 21 amino acids, and a β-chain (also referred to as B-chain), i.e., a peptide consisting of 30 amino acids. Insulin is involved in sugar, amino acid, and fat metabolism, and it is physiologically important in the hypoglycemic effect. Diabetes is caused by insufficient insulin secretion due to decrease in or the functional deterioration of beta cells or due to insufficient insulin action in peripheral tissues. Therefore, the measurement of blood insulin concentration reflecting the insulin secretory function of beta cells is a useful index for the diagnosis and understanding of the clinical condition of diabetes and determination of the cause of abnormal glucose tolerance.

On the other hand, insulin replacement therapy is an important means of diabetes treatment. The therapy is performed by administering conventional bovine and porcine insulin, human insulin acquired by gene recombination, and insulin analog formulations

(hereinafter also referred to as insulin analogs) acquired by a change (substitution, deletion, addition, insertion) in an amino acid sequence of human insulin or by modification of a portion of constituent amino acid with fatty acid. To determine precise clinical effect of such insulin replacement therapy, it is desired to distinguish endogenous insulin produced in the body by a diabetic patient from exogenous insulin administered into the body from the outside so as to specifically measure endogenous insulin present in the human body.

The followings are disclosures related to a human insulin assay using a monoclonal antibody.

Patent Literature 1 discloses a method of quantitating human insulin in accordance with an enzyme-linked immunosorbent assay (hereinafter also referred to as ELISA). This assay uses an anti-human insulin monoclonal antibody bound to an insoluble carrier and an anti-human insulin monoclonal antibody recognizing an epitope not competing with an epitope of the antibody and labeled with an enzyme. Patent Literature 1 has no description about reactivity with insulin derived from animal species other than human including porcine insulin and insulin analogs and it is unclear whether human insulin can specifically be measured.

Patent Literature 2 discloses a method of quantitating human insulin in accordance with a particle agglutination immunoassay. This assay uses two mouse-produced anti-human insulin monoclonal antibodies that have different recognition sites and are bound to insoluble carriers. Although the two mouse-produced anti-human insulin monoclonal antibodies are described as being produced based on a method described in Patent Literature 3, Patent Literature 3 describes that a mouse-produced anti-human insulin monoclonal antibody is produced by using porcine insulin as an immunogen. Patent Literature 2 also describes that the reactivity to standard human insulin is the same in the particle agglutination immunoassay between when a polyclonal antibody purified from a guinea-pig-produced porcine insulin antiserum is used and when the two mouse-produced anti-human insulin monoclonal antibodies are used.

Patent Literature 3 discloses a monoclonal antibody to porcine insulin or human insulin, a production method thereof, and a radioimmunoassay (hereinafter also referred to as RIA) using the monoclonal antibodies. Patent Literature 3 mentions that (1) antiserum acquired by immunizing an animal such as guinea pig with bovine insulin or porcine insulin is used for measuring human insulin, that (2) when bovine insulin is used as an immunogen, it is difficult to acquire a monoclonal antibody reactive to human insulin as compared to the case of using porcine insulin or human insulin as an immunogen, and that (3) since porcine insulin, unlike bovine insulin, is only different in amino acid at B-chain C-terminal, a monoclonal antibody reactive to human insulin can be acquired by using porcine insulin as an immunogen, and discloses that a monoclonal antibody is acquired by using porcine insulin as an immunogen and that the acquired monoclonal antibody preferably cross-reacts with porcine insulin and human insulin.

Considering Patent Literatures 2 and 3, it is deduced that porcine insulin is used as immunogens for the two mouse-produced anti-human insulin monoclonal antibodies produced based on the method of Patent Literature 3 and described in Patent Literature 2, and such an antibody should react with porcine insulin.

Patent Literatures 1 and 2 both describe methods of measuring human insulin by using a plurality of monoclonal antibodies having different recognition sites for human insulin and include no idea of using an antibody at least nonreactive with porcine insulin to specifically measure human insulin.

Non Patent Literatures 1 to 3 report reaction specificity of commercially available human insulin assay reagents (cross-reactivity (rates) of porcine insulin and insulin analogs to human insulin).

Non Patent Literature 1 discloses that one of two commercial reagents has cross-reactivity of 19.2 % with porcine insulin and cross-reactivity of 0.02 % with insulin lispro, which is an insulin analog, and that the other reagent has cross-reactivity of 100 % with porcine insulin and cross-reactivity of 75 % with insulin lispro.

Non Patent Literature 2 discloses that 16 reagents of 26 commercial reagents have cross-reactivity of 19.2 % to 450 % with porcine insulin and that 8 reagents have cross-reactivity of less than 0.1 % to 100 % with insulin lispro.

Non Patent Literature 3 discloses that measurement of a dilution series of insulin analogs in 6 commercial reagents revealed that average cross-reactivity of one reagent with insulin aspart, insulin glargine, and insulin lispro was less than 0.7 % and average cross-reactivity of the other five reagents with the three insulin analogs was less than 3.6 % to 143 %. However, the literatures have no disclosure of cross-reactivity of these reagents with porcine insulin.

As described above, a reagent having no cross-reactivity with porcine insulin does not exist in commercial reagents for human insulin assay. One commercial reagent has cross-reactivity of less than 0.7 % with a plurality of insulin analogs and one commercial reagent has cross-reactivity of less than 10 %. The commercial reagent of Non Patent Literature 3 having cross-reactivity of less than 0.7 % with a plurality of insulin analogs is the commercial reagent of Non Patent Literatures 1 and 2 having cross-reactivity of 19.2 % with porcine insulin.

CITATION LIST

PATENT LITERATURE

Patent Literature 1: Japanese Laid-Open Patent Publication No. H01-148962
Patent Literature 2: Japanese Laid-Open Patent Publication No. H03-118472
Patent Literature 3: Japanese Laid-Open Patent Publication No. S60-188327

NON PATENT LITERATURE

Non Patent Literature 1: Clinical chemistry, 47[3] (2001) P.602-5
Non Patent Literature 2: Clinical laboratory, 49[3-4] (2003) P.113-21
Non Patent Literature 3: Clinical chemistry, 50[1] (2004) P.257-9

SUMMARY OF INVENTION

TECHNICAL PROBLEM

The present invention provides an anti-human insulin antibody nonreactive with porcine insulin and specifically reactive with human insulin, and a human insulin-specific assay and an assay reagent using the antibody.

SOLUTION TO PROBLEM

As a result of extensive research, the inventors discovered an anti-human insulin antibody nonreactive with porcine insulin, bovine insulin, proinsulin, and insulin analogs and specifically reactive with human insulin when the anti-human insulin antibody was screened while human insulin maintains the conformation thereof in a solution rather than being immobilized to a solid phase, and further discovered that the specific antibody can be used in an immunoassay to distinguish, and accurately measure, human insulin from porcine insulin etc., thereby completing the present invention. Therefore, the present invention includes the followings:

[1] An anti-human insulin antibody having the following properties (a) and (b):

a) the antibody reacts with human insulin, and
b) the antibody does not react with porcine insulin.

[2] The anti-human insulin antibody of claim 1, further having one or more of the following properties:

c) the antibody does not react with bovine insulin,
d) the antibody does not react with canine insulin,

e) the antibody does not react with rabbit insulin,

f) the antibody does not react with proinsulin,

g) the antibody does not react with an insulin analog, and

h) the antibody does not react with a peptide fragment consisting of a sequence RGFFYTPKT (SEQ ID NO. 1).

[3] The anti-human insulin antibody of [2], wherein the insulin analog is selected from a group consisting of insulin lispro, insulin aspart, insulin glargine, insulin detemir, and insulin glulisine.

[4] The anti-human insulin antibody of any one of [1] to [3], further having the following properties:

(i) the antibody recognizes a conformation of a β-chain C-terminal RGFFYTPKT region in a human insulin molecule.

[5] The anti-human insulin antibody of [4], wherein the conformation of the β-chain C-terminal RGFFYTPKT region in a human insulin molecule is a conformation achievable in the following solution:

0.01 M HEPES (pH 8.5), 0.15 M sodium chloride, 3 mM EDTA, and 0.005 % Surfactant P20.

[6] The anti-human insulin antibody of any one of [1] to [5], wherein the anti-human insulin antibody is a monoclonal antibody.

[7] The anti-human insulin antibody of [6], wherein the anti-human insulin antibody is produced by a hybridoma of the accession number FERM BP-11314.

[8] The anti-human insulin antibody of [6], wherein the anti-human insulin antibody is capable of recognizing an epitope identical to an epitope recognized by a monoclonal antibody produced by the hybridoma of the accession number FERM BP-11314.

[9] A human insulin assay comprising a step of bringing the antibody of any one of claims 1 to 8 into contact with a biological sample to detect a complex of the antibody and human insulin formed by the contact.

[10] The human insulin assay of [9], wherein the antibody of any one of [1] to [8] is labeled with a detectable labeling material.

[11] A human insulin assay using the following two antibodies:

1) the anti-human insulin antibody of any one of [1] to [8], and

2) an antibody A having a property of reacting at least with human insulin.

[12] A human insulin assay using the following two antibodies:

1) the anti-human insulin antibody of any one of [1] to [8], and

2) an antibody B having a property of specifically recognizing the antibody of 1).

[13] The human insulin assay of [11] or [12], wherein the both antibodies of 1) and 2) are monoclonal antibodies.

[14] The human insulin assay of [11] or [12], wherein the antibody of 1) is a monoclonal antibody, and wherein the antibody of 2) is a polyclonal antibody.

[15] The human insulin assay of any one of [11] to [14], wherein the antibody of 1) and/or the antibody of 2) are immobilized to a solid phase.

[16] The human insulin assay of [15], wherein the solid phase is latex, and wherein insulin is assayed by a latex immunoagglutination assay.

[17] The human insulin assay of [16], wherein the antibody of 1) is immobilized to a solid phase, wherein the antibody of 2) is labeled with a labeling material, and wherein insulin is assayed by ELISA or immunochromatography.

[18] An exogenous insulin assay comprising the steps of:

(1) obtaining a total concentration of human insulin and exogenous insulin;

(2) obtaining a human insulin concentration with the insulin assay of any one of [9] to [17]; and

(3) obtaining an exogenous insulin concentration by subtracting the concentration obtained at (2) from the concentration obtained at (1).

[19] An insulin assay reagent, wherein the insulin assay reagent uses the antibody of any one of [1] to [8].

[20] An insulin assay reagent using the following two antibodies:

1) the anti-human insulin antibody of any one of [1] to [8], and

2) an antibody A having a property of reacting at least with human insulin.

[21] An insulin assay reagent using the following two antibodies:

1) the anti-human insulin antibody of any one of [1] to [8], and
2) an antibody B having a property of specifically recognizing the antibody of 1).

[22] The insulin assay reagent of [20] or [21], wherein the both antibodies of 1) and 2) are monoclonal antibodies.
[23] The insulin assay reagent of [20] or [21], wherein the antibody of 1) is a monoclonal antibody, and wherein the antibody of 2) is a polyclonal antibody.
[24] The human insulin assay reagent of any one of [20] to [23], wherein the antibody of 1) and/or the antibody of 2) are immobilized to a solid phase.
[25] The human insulin assay reagent of [24], wherein the solid phase is latex, and wherein insulin is assayed by a latex immunoagglutination assay.
[26] The human insulin assay reagent of [24], wherein the antibody of 1) is immobilized to a solid phase, wherein the antibody of 2) is labeled with a labeling material, and wherein insulin is assayed by ELISA or immunochromatography.
[27] An exogenous insulin assay kit including the following assay reagents:

(1) a reagent for measuring a total insulin concentration of human insulin and exogenous insulin, and
(2) the human insulin assay reagent of any one of [19] to [26].

ADVANTAGEOUS EFFECTS OF INVENTION

With the present invention, human insulin can accurately be assayed without being affected by porcine insulin, bovine insulin, proinsulin, and insulin analogs. Since human insulin secreted from the beta cells of a patient alone can accurately be assayed from a diabetic patient under the insulin replacement therapy subjected to the administration of porcine insulin, insulin analogs, etc., with the present invention, a clinical condition of a diabetic patient can accurately be understood.

Only exogenousinsulin such as insulin derived from animal species other than human and insulin analogs can be assayed from i) an assay result of a total amount (total concentration) of human insulin, insulin derived from animal species other than human, and insulin analogs from an assay using an anti-human insulin antibody cross-reactive with human insulin as well as insulin derived from animal species other than human and insulin analogs used in the insulin replacement therapy, and ii) an assay result of only human insulin from the assay using the antibody of the present invention.

BRIEF DESCRIPTION OF DRAWINGS

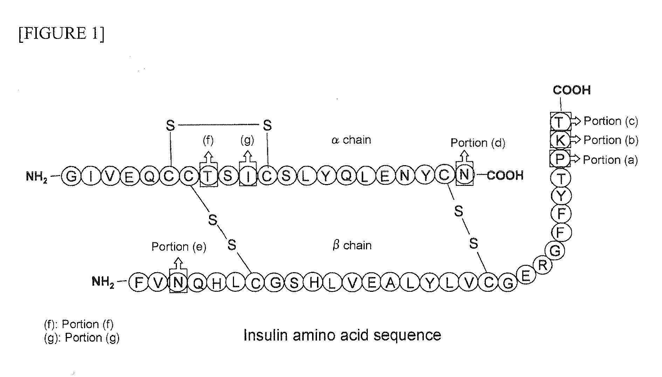

[Fig. 1] Fig. 1 is a scheme of the amino acid sequence of human insulin. In Fig. 1, (a) through (g) are indicative of the variations in the amino acid sequence of human insulin from insulin derived from animal species other than human (porcine insulin, bovine insulin, rabbit insulin, and canine insulin) and insulin analogs (insulin lispro, insulin aspart, insulin glargine, insulin detemir, and insulin glulisine) whose reactivity with antibodies of the present invention was assayed. Alphabetic characters in the circles of Fig. 1 denote amino acids represented by one character.

<Insulin Derived from Animal Species>

Porcine insulin: portion (c) is "A" instead of "T".
Bovine insulin: portion (c) is "A" instead of "T", portion (f) is "A" instead of "T", and portion (g) is "V" instead of "I".
Rabbit insulin: portion (c) is "S" instead of "T".
Canine insulin is the same as porcine insulin.

<Insulin Analogs>

Insulin lispro: portions (a) and (b) are "K-P" instead of "P-K."
Insulin aspart: portion (a) is "D" instead of "P".

Insulin glargine: portion (d) is "G" instead of "N" and "RR" is added to "T" of portion (c).
Insulin detemir: "T" is absent from portion (c) and myristic acid ($C_{14}H_{28}O_2$) is added to "K" of portion (b).
Insulin glulisine: portion (b) is "E" instead of "K" and portion (e) is "K" instead of "N".

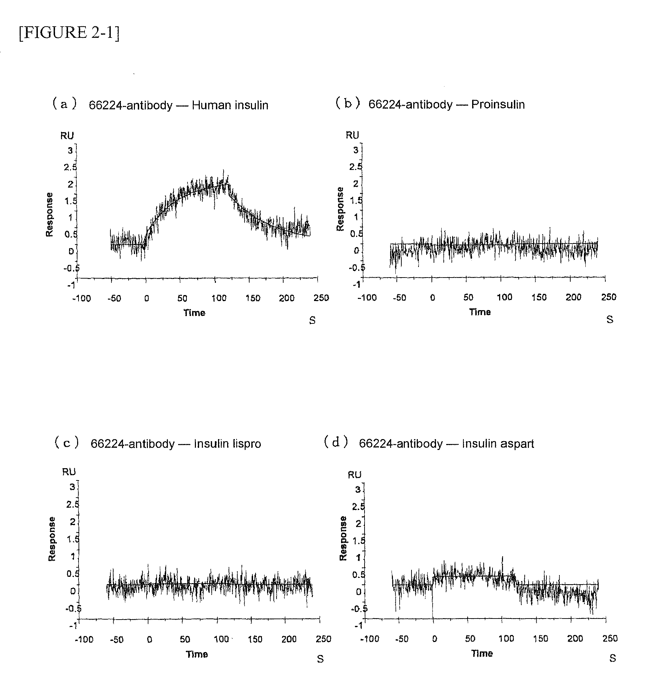

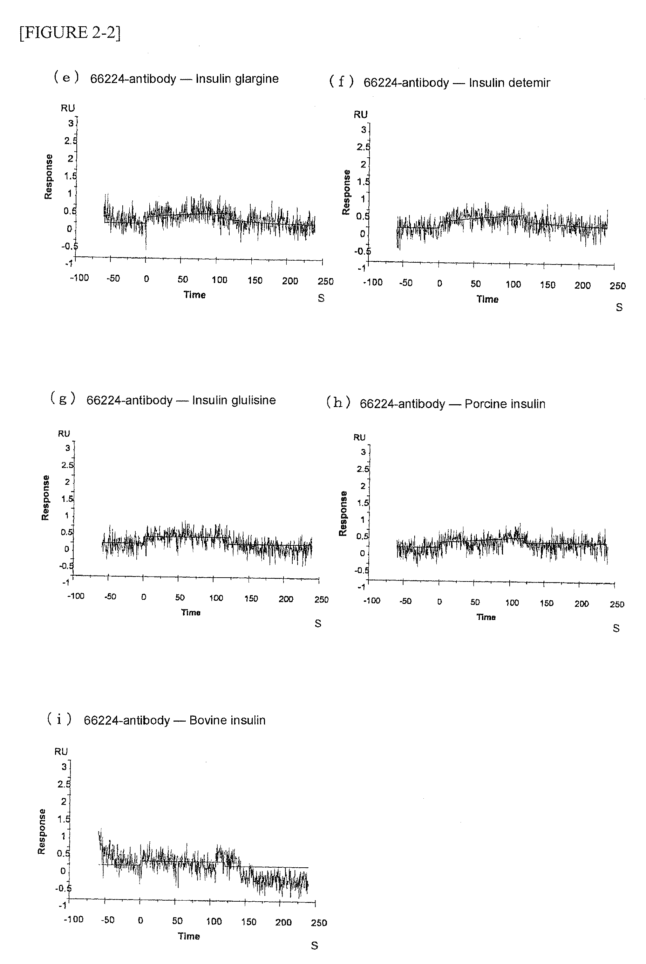

[Fig. 2-1] Fig. 2-1 is a diagram of the results of a test using Biacore (registered trademark) T100 for examining reactivity of the 66224-antibody with human insulin, proinsulin, various insulin analogs, porcine insulin, and bovine insulin. In Fig. 2-1, (a),(b), (c), and (d) are results for human insulin, proinsulin, insulin lispro, and insulin aspart, respectively.

[Fig. 2-2] Fig. 2-2 is the same as above. In Fig. 2-2, (e), (f), (g), (h), and (i) are the results for insulin glargine, insulin detemir, insulin glulisine, porcine insulin, and bovine insulin, respectively.

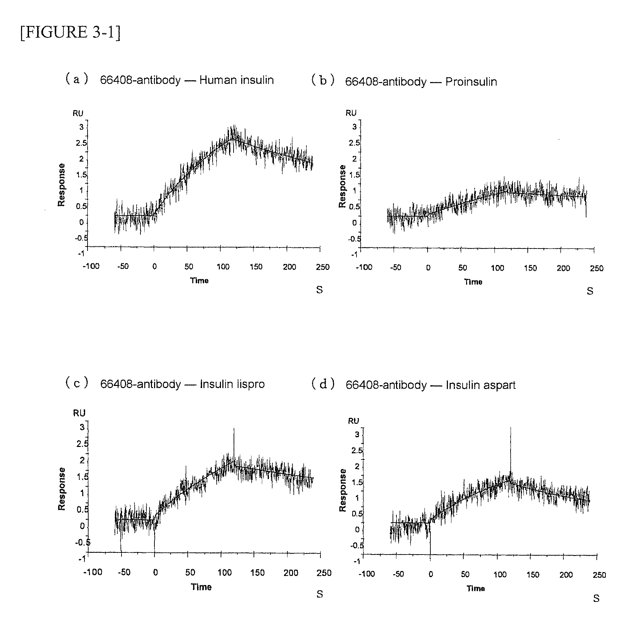

[Fig. 3-1] Fig. 3-1 is a diagram of the results of a test using Biacore (registered trademark) T100 for examining reactivity of the 66408-antibody with human insulin, proinsulin, various insulin analogs, porcine insulin, and bovine insulin. In Fig. 3-1, (a), (b), (c), and (d) are results for human insulin, proinsulin, insulin lispro, and insulin aspart, respectively.

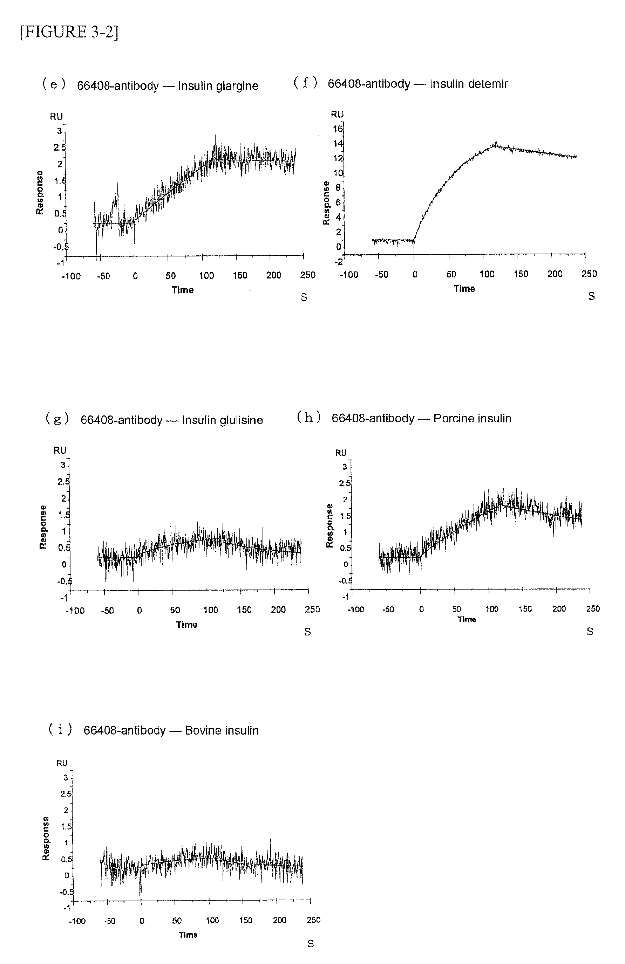

[Fig. 3-2] Fig. 3-2 is the same as above. In Fig. 3-2, (e), (f), (g), (h), and (i) are the results for insulin glargine, insulin detemir, insulin glulisine, porcine insulin, and bovine insulin, respectively.

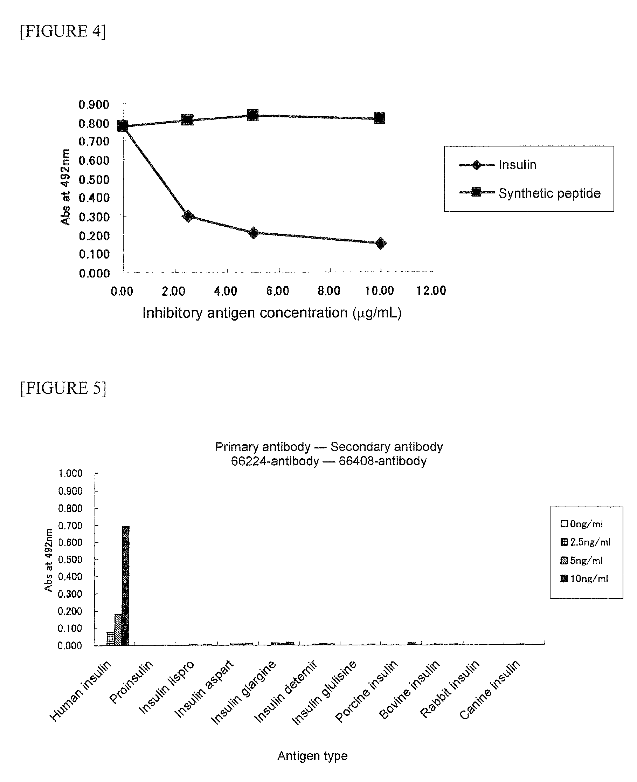

[Fig. 4] Fig. 4 is a diagram of the result of a test using competitive ELISA for examining reactivity of the 66224-antibody with a peptide fragment consisting of the sequence "RGFFYTPKT" (SEQ ID NO. 1) of the C-terminal region of the human insulin β-chain (the amino acid sequence of the peptide is different from porcine insulin only in that the C-terminal amino acid is "T"(porcine insulin has the C-terminal amino acid "A")).

[Fig. 5] Fig. 5 is a diagram of the result of a test using sandwich ELISA for examining reactivity with human insulin, proinsulin, various insulin analogs, porcine insulin, bovine insulin, rabbit insulin, and canine insulin, using the 66224-antibody and 66408-antibody as the primary and secondary antibodies, respectively, with the primary antibody solid-phased on a plate.

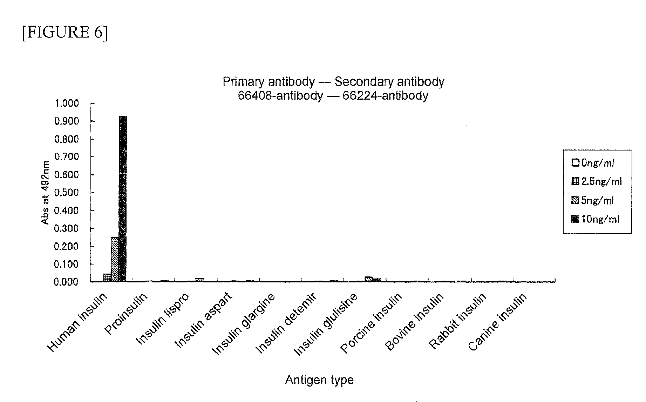

[Fig. 6] Fig. 6 is a diagram of the result of a test using sandwich ELISA for examining reactivity with human insulin, proinsulin, various insulin analogs, porcine insulin, bovine insulin, rabbit insulin, and canine insulin, using the 66408-antibody and 66224-antibody as the primary and secondary antibodies, respectively, with the primary antibody solid-phased on a plate.

## DESCRIPTION OF EMBODIMENTS

When a compound "reacts with", "is reactive with", "has reactivity with", and "bounds to" an antibody or an antibody "recognizes" a compound in this description, these expressions have meanings normally used in the art of the present invention and are used synonymously. However, these expressions must be construed in the broadest sense, including other expressions having the same meanings used in the art of the present invention such as "has affinity for", without being limited to these exemplifications. Whether an antibody "reacts with" a compound can be confirmed by solid-phase antigen ELISA, competitive ELISA, and sandwich ELISA described later and well known to those skilled in the art and can also be identified by a method utilizing the principle of surface plasmon resonance (SPR method). The SPR method can be performed using devices, sensors, and reagents commercially available under the name of Biacore (registered trademark).

In this description, stating that an antibody of the present invention "is nonreactive with / does not react with" a compound suggests that the antibody of the present invention is substantially nonreactive with the compound. Stating "substantially nonreactive / substantially does not react" suggests that when Biacore (registered trademark) T100 is used for immobilizing the antibody of the present invention to assay reactivity with a tested compound based, for example, on the SPR method, the reactivity between the antibody of the present invention and the tested compound is not significantly increased relative to the reactivity in the control experiment (test in the absence of the tested compound). Needless to say, it can be confirmed that an antibody is "substantially nonreactive" with a compound by a method/means well known to those skilled in the art, in addition to the SPR method.

In this description, "cross-reaction (cross-reactivity)" suggests a property of an antibody not only specifically (selectively) reacting only with (binding only to) an original antigen but also nonspecifically reacting with (binding to) substance (hereinafter also referred to as cross-reactive material) with a chemical structure similar to the original antigen. An extent of the nonspecific reaction (binding) between the antigen and the cross-reactive material is indicated, for example, by a rate to the reaction (binding) between the antigen and the original antigen and represented as cross-reactivity or a cross-reaction rate.

In this description, an "insoluble carrier" may be expressed as a "solid phase". Although physically or chemically supporting an antigen or antibody with an insoluble carrier or the supporting state may be described as "immobilizing," "immobilized," and "solid-phased", these expressions include other expressions with the same meanings used in the art of the present invention such as "sensitization" and "adsorption".

In this description, the term "detection" or "measurement" must be construed in the broadest sense including the existence proof and/or quantitation of insulin and must not be construed as limiting in any sense.

In this description, "exogenous insulin" suggests insulin administered into the body from the outside for diabetes treatment as opposed to so-called "endogenous insulin" produced in the human body and specifically suggests insulin derived from animal species other than human and/or insulin analogs.

An anti-human insulin antibody of the present invention is an antibody specifically reactive with human insulin and nonreactive with porcine insulin. The anti-human insulin antibody of the present invention may also be nonreactive with any one or more of bovine insulin, canine insulin, rabbit insulin, proinsulin, and insulin analogs. The insulin analogs include insulin lispro, insulin aspart, insulin glargine, insulin detemir, and insulin glulisine. The insulin analogs are also referred to as "insulin analog formulations" as described above.

The anti-human insulin antibody of the present invention is desirably nonreactive with a peptide fragment consisting of the sequence "RGFFYTPKT" (SEQ ID NO. 1) of the C-terminal region of the human insulin β-chain in addition to the reactivity described above. Human insulin and porcine insulin are different from each other only in that the β-chain C-terminal amino acid is "T" or "A". Therefore, to acquire an antibody specifically reactive with human insulin and nonreactive with porcine insulin, an antibody may be selected that is specifically reactive with a peptide fragment including the amino acid sequence of the β-chain C-terminal region of human insulin; however, by selecting an antibody reactive with human insulin, nonreactive with porcine insulin, and nonreactive with the peptide fragment including the amino acid sequence of the C-terminal region of the human insulinβ-chain, an antibody can be acquired that recognizes the conformation of human insulin involving a sequence that embraces the amino acid different between human insulin and porcine insulin, thereby desirably ensuring higher specificity.

The anti-human insulin antibody of the present invention may be a monoclonal antibody. Specifically, a monoclonal antibody (66224-antibody) produced by a hybridoma 66224 (FERM BP-11314) can be cited. The anti-human insulin antibody of the present invention also includes an antibody capable of recognizing an epitope identical to an epitope recognized by the monoclonal antibody produced by the hybridoma of FERM BP-11314. As in the case of the 66226-antibody described in PCT/JP2010/62261 (monoclonal antibody produced by the hybridoma of International Deposition No. FERM BP-11234), an antibody recognizing a conformation of insulin bound to an anti-human insulin antibody (hereinafter also referred to as complex insulin) but nonreactive with porcine insulin may also be used in the same way as the antibody of the present invention. Such an antibody may be used as an antibody having the same characteristics as the antibody of the present invention by combining with an antibody whose complex insulin forms different conformations when bound to human insulin and porcine insulin.

The antibodies of the present invention can be easily produced by dissolving human insulin as an antigen (immunogen) in solvent, such as phosphate-buffered saline, and administering this solution to immunize an animal other than human (hereinafter also simply referred to as an animal in description related to antibody acquisition). Although insulin utilized as an antigen may be the entire insulin molecule or a portion thereof, the entire insulin molecule is preferably utilized to maintain a conformation in human insulin involved with the amino acid sequence of the C-terminal region of the human insulin β-chain so as to acquire an antibody with higher specificity as described above. After adding an appropriate adjuvant to the solution to form an emulsion as required, the immunization may be performed using the emulsion. The adjuvant may be a widely used adjuvant, such as water-in-oil emulsion, water-in-oil-in-water emulsion, oil-in-water emulsion, liposome, or aluminum hydroxide gel as well as a protein or peptidic substance derived from biogenic components. For example, Freund's incomplete or complete adjuvant can be used in a preferred manner. Although not particularly limited, it is desired that the administration route, administered dose, and administration time of the adjuvant are appropriately selected such that a desired immune response can be enhanced in an animal to be immunized by the antigen.

Although the choice of the animal used for the immunization is not particularly limited, it is preferably a mammal and can be a mouse, rat, bovine, rabbit, goat, and sheep, although a mouse is more preferred. The animal may be immunized in accordance with a common technique, e.g., the immunization can be achieved by subcutaneously, intracutaneously, intravenously, or intraperitoneally injecting the animal with a solution of an antigen, preferably a mixture with the adjuvant. Since an immune response is generally different depending on the type and strain of an animal to be immunized, it is desirable that an immunization schedule is appropriately set depending on the animal to be used. Preferably, the antigen administration is repeated several times after the initial immunization.

The following operations are subsequently performed to acquire a monoclonal antibody, but these operations are not limitations. A method of producing a monoclonal antibody itself can be performed in conformity with a method described, for example, in Antibodies, A Laboratory Manual (Cold Spring Harbor Laboratory Press, (1988)).

After the final immunization, the hybridoma can be produced by extracting spleen or lymph node cells, which are antibody-producing cells, from an immunized animal and by fusing these cells with proliferative myeloma cells. It is preferred that cells having high antibody-producing ability (quantitative and qualitative) be used for the cell fusion and that the myeloma cells be compatible with the animal from which the antibody-producing cells to be fused are derived. The cell fusion can be performed in accordance with a method known in the art, and a polyethylene glycol method, a method using Sendai virus, or a method utilizing electric current can be employed. The acquired hybridoma can be proliferated in accordance with a known method, and the desired hybridoma can be selected while identifying the property of the produced antibody. The hybridoma can be cloned by a known method such as a limiting dilution or soft agar method.

The hybridoma can efficiently and effectively be selected, considering the condition under which the produced antibody is actually used in the assay. As a standard example, it may be mentioned that the hybridoma can be acquired by selecting a hybridoma that produces an antibody reactive with insulin through ELISA, RIA, or a method using Biacore (registered trademark). In particular, the solid-phase antigen ELISA, initially reacting an antibody in the culture supernatant of a hybridoma with solid-phased human insulin on a plate etc., and subsequently reacting labeled anti-IgG antibodies, is used for selecting a hybridoma that produces a monoclonal antibody that is highly reactive with human insulin.

For example, Biacore (registered trademark) T100 can also be used for confirming the reactivity with porcine insulin, bovine insulin, proinsulin, insulin analogs (insulin lispro, insulin aspart, insulin glargine, insulin detemir, and insulin glulisine) and selecting a hybridoma with desired reactivity (specificity) to make sure that a hybridoma producing the anti-human insulin antibody of the present invention is selected. It is deduced that in the confirmation of the reactivity one can easily narrow the anti-human insulin monoclonal antibody recognizing the conformation of human insulin down by confirming the reactivity with human insulin maintaining the conformation thereof in a solution without being immobilized to a solid phase.

By producing a peptide fragment of human insulin including an amino acid sequence of the C-terminal region of the human insulin β-chain different from porcine insulin only in the β-chain C-terminal amino acid and by selecting a hybridoma producing a monoclonal antibody nonreactive with the peptide fragment, a hybridoma can be acquired that produces a monoclonal antibody recognizing the sequence in the conformation of human insulin rather than a primary structure of the sequence. Although a peptide fragment consisting of a sequence "RGFFYTPKT" of the C-terminal region of the human insulin β-chain may preferably be used as the peptide fragment, any peptide fragment of the human insulin C-terminal region may be used as long as the peptide fragment contains the C-terminal amino acid of the human insulin β-chain and has a length at least recognizable for the antibody. The number of amino acids of the peptide is preferably five or more.

A method of screening the hybridoma (antibody) of the present invention is summarized as follows in accordance with examples described later.

Primary Screening: Solid-phase antigen ELISA is performed to confirm reactivity to human insulin and select positive wells.

Secondary Screening: Competitive ELISA of human insulin is performed to reconfirm that the antibody is reactive with human insulin and select positive wells.

Tertiary screening: A reactivity assay using Biacore (registered trademark) is used for selecting wells having specific reactivity to human insulin and having no cross-reactivity to insulin derived from animal species other than human, proinsulin, and insulin analogs.

Quaternary screening: Competitive ELISA with a peptide fragment consisting of the sequence "RGFFYTPKT" of the C-terminal region of the human insulin β-chain is performed to select wells having no reactivity to the peptide fragment and having high reactivity to human insulin.

While not wishing to be bound by any particular theory, the inventor deduces one reason of the completion of the present invention as follows.

In a conventional screening, human insulin is directly or indirectly soild-phased or labeled and, therefore, a portion of the original conformation of human insulin may possibly be lost. As described above, porcine insulin and human insulin have a structural difference only in the β-chain C-terminal amino acid and, in such a case, a slight change in the conformation may possibly have significant effect on an epitope determination of an antibody. In the present invention, as described above, Biacore (registered trademark) is used for performing the screening while the conformation of human insulin is maintained and competitive ELISA with a peptide fragment of the C-terminal region of the human insulin β-chain is used for performing the screening with the conformation intentionally lost so as to select an antibody having higher specificity.

From the entire description herein, those skilled in the art will be able to understand that a hybridoma producing the antibody of the present invention can be screened by confirming at least the reactivity to human insulin and porcine insulin in the reactivity assay using Biacore (registered trademark).

A monoclonal antibody having a desired property can be produced by the mass cultivation of the hybridoma selected in this manner. A method of mass cultivation is not particularly limited and can include, e.g., a method of producing the monoclonal antibody in culture media by cultivating the hybridoma in appropriate culture media and a method of producing the antibody in ascites by injecting for proliferation the hybridoma into the abdominal cavity of a mammal. The monoclonal antibody can be purified by appropriately combining anion exchange chromatography, affinity chromatography, the ammonium sulfate fractionation method, the PEG fractionation method, and the ethanol fractionation method, for example.

The antibodies of the present invention can be whole antibody molecules as well as functional fragments having antigen-antibody reaction activity. The antibodies can be those acquired through immunization of animals, by a gene recombination technique, or chimeric antibodies. The functional fragments of antibodies include F(ab')$_2$ and Fab', and these functional fragments can be produced by processing the antibodies acquired as described above with a proteolytic enzyme (e.g., pepsin or papain).

The antibody of the present invention may be immobilized on an insoluble carrier or labeled with a well-known and commonly used labeling material, which we will describe later. We may refer to them as "immobilized (solid phase) antibodies" and labeled antibodies, respectively. Such immobilized or labeled antibodies are included in the scope of the present invention. For example, an immobilized antibody can be produced by causing an insoluble carrier to physically adsorb or chemically bind to the antibody of the present invention (a suitable spacer may exist in between them). The insoluble carrier can be made of a polymer base material such as a polystyrene resin, an inorganic base material such as glass, and a polysaccharide base material such as cellulose and agarose, and the shape is not particularly limited and can be selected arbitrarily. For example, the insoluble carrier may be in the shape of a plate (e.g., microplate and membrane), beads, particles (e.g., latex particles), or a cylinder (e.g., test tube).

Labeling materials for producing antibodies include for example, enzymes, fluorescent materials, chemiluminescent materials, biotin, avidin, or radio isotopes, colloidal gold particles, and colored latex. Labeling materials can be bound to the antibodies by conventional methods, such as glutaraldehyde method, maleimide method, pyridyl disulfide method, and periodic acid method. However, the types of immobilized or labeled antibody and the producing methods are not limited to those described above. For example, when an enzyme such as peroxidase or alkaline phosphatase is used as a labeling material, the enzymatic activity may be assayed using a specific substrate of the enzyme, e.g., 1,2-phenylenediamine (OPD) or 3,3',5,5'-tetramethylbenzidine for horseradish peroxidase (HRP), and p-nitrophenyl phosphate for ALP. When biotin is used as the labeling material, at least avidin or enzyme-modified avidin is normally used in the reaction.

The anti-human insulin antibody of the present invention can be used in combination with A: an anti-human insulin antibody at least reactive with human insulin (hereinafter also referred to as an antibody A) or B: an antibody specifically recognizing the anti-human insulin antibody of the present invention (hereinafter also referred to as an antibody B).

The antibody A is not particularly limited as long as the antibody has reactivity with human insulin and may have cross-reactivity with any one of proinsulin, insulin analogs (insulin lispro, insulin aspart, insulin glargine, insulin detemir, and insulin glulisine), porcine insulin, and bovine insulin. The antibody A may be a monoclonal antibody or a polyclonal antibody as long as the antibody is reactive with human insulin and specifically includes the monoclonal antibody (66408-antibody) produced by the hybridoma 66408 (FERM BP-11315) and the monoclonal antibody produced by the hybridoma 66221 (FERM BP-11314) in the case of the monoclonal antibody. The antibody A may be a whole antibody molecule as well as a functional fragment of an antibody reactive with human insulin. A human insulin recognition site of the antibody A is not necessarily completely independent of the human insulin recognition site of the anti-human insulin antibody of the present invention on the condition that an assay and an assay reagent of the present invention can be configured by combining the antibody A and the antibody of the present invention.

The antibody B refers to an antibody used in an indirect detection system such as a so-called double antibody method or used for sensitization and may be any antibody specifically reactive with the anti-human insulin antibody of the present invention and may be a monoclonal antibody or a polyclonal antibody. The antibody B may be a whole antibody molecule as well as a functional fragment of an antibody reactive with the anti-human insulin antibody of the present invention. If the antibody of the present invention is a mouse-produced monoclonal antibody, the antibody B can include an anti-mouse IgG antibody.

If the antibody of the present invention is used in combination with the antibody A, one or more of the antibody of the present invention and the antibody A can be labeled with the labeling material or may be immobilized to the insoluble carrier before use. Specific forms in this case include sandwich ELISA and particle agglutination immunoassay.

The form of an assay reagent (kit) provided by the present invention is not particularly limited as long as the reagent is capable of assaying human insulin. Well-known label immunoassays, i.e., sandwich ELISA and immunochromatography, and a well-known particle agglutination immunoassay, i.e., latex immunoagglutination assay (hereinafter also referred to as LTIA), will hereafter be described as examples.

<Label Immunoassay: Sandwich ELISA>

The forms of the assay reagent (kit) for detecting human insulin present in a sample may be the following two forms A and B requiring elements (a) and (b):

A. (a) a solid phase with the anti-human insulin antibody of the present invention immobilized, and (b) an antibody A labeled with a labeling material and at least having reactivity with human insulin (hereinafter also referred to as a labeled antibody A); and

B. (a) the anti-human insulin antibody of the present invention labeled with a labeling material, and (b) a solid phase with an antibody A at least reactive with human insulin immobilized.

The antibody immobilized to a solid phase captures human insulin in a sample to form a complex on the solid phase. The antibody labeled with the labeling material binds to the captured human insulin to form a sandwich with the complex. The human insulin in the sample can be assayed by measuring an amount of the labeling material by a method suitable for the labeling material. With regard to specific methods for configuring the assay reagent (kit), such as a method for immobilizing the antibody and a method for labeling the antibody with the labeling material, techniques well-known to those skilled in the art can be used without particular limitation, in addition to those described herein. This configuration can preferably be formed as a homogeneous assay system or a heterogeneous assay system.

<Label Immunoassay: Immunochromatography>

Typical immunochromatography is configured such that in order of distance from the edge in the direction of spread of a test sample solution on a sheet-shaped solid phase such as a membrane, the test sample solution continuously moves because of capillary phenomenon through test pieces equipped with "1. a sample loading site", "2. a labeled reagent site holding the labeled antibody A (labeled with colloidal gold or colored latex) in a spreadable manner on the membrane", and "3. a capture reagent site with the antibody of the present invention immobilized for capturing the complex formed by the labeled antibody A and human insulin".

In particular, when a predetermined quantity of a test sample containing insulin is added to the sample loading site, the sample infiltrates the labeled reagent site due to the capillary phenomenon, and the insulin binds to the labeled antibody A to form a complex of insulin and the labeled antibody A. The complex continues spreading and moving on the membrane, and when infiltrating into the capture reagent site on the membrane, which contains the antibody of the present invention, the complex is captured by the capture reagent immobilized on the solid-phase to form a ternary complex of capture reagent-insulin-labeled antibody A at the capture reagent site. The presence of insulin can be detected by detecting the labeled reagent by a method of your choice, e.g., detecting the appearance of agglutination (agglutination image/picture) in the case of a label that can be visualized, such as colloidal gold, and detecting the chromogenic reaction due to addition of a substrate in case of enzyme.

<Particle Agglutination Immunoassay: LTIA>

The forms of the assay reagent (kit) for detecting human insulin present in a sample may be the following four forms A to D requiring elements (a) and (b), or only (a):

A. (a) latex particles with the anti-human insulin antibody of the present invention immobilized and (b) latex particles with an antibody A at least reactive with human insulin immobilized;
B. (a) latex particles with the anti-human insulin antibody of the present invention immobilized and (b) the antibody A at least reactive with human insulin;
C. (a) the anti-human insulin antibody of the present invention and (b) latex particles with the antibody A at least reactive with human insulin immobilized; and
D. (a) latex particles with both the anti-human insulin antibody of the present invention and the antibody A at least reactive with human insulin immobilized.

These assay reagents (kits) can be used particularly in LTIA in a preferred manner. The latex particles used in A to D can be selected appropriately in terms of particle diameter and type in order to achieve the desired capability, such as enhanced sensitivity. The latex particles may be those suitable for carrying an antigen or antibody. For example, the latex particles may be of polystyrene, styrene-sulfonic acid (sulfonate) copolymer, styrene-methacrylic acid copolymer, acrylonitrile-butadiene-styrene copolymer, vinyl chloride-acrylic ester copolymer, or vinyl acetate-acrylic acid ester copolymer. Although the shape of the latex particles is not particularly limited, it is preferable that an average particle diameter is defined such that the produced aggregate, as a result of the agglutination reaction between the antibody (or antigen) on the latex particle surface and the analyte, has a size sufficient to be visibly or optically detected. When a transmission electron microscope is used, the average particle diameter is preferably 0.02 to 1.6 $\mu$m and particularly 0.03 to 0.5 $\mu$m. Particles made of metallic colloid, gelatin, liposome, microcapsule, silica, alumina, carbon black, metallic compound, metal, ceramics, or magnetic material can be used instead of the latex particles.

The reagent of LTIA used in clinical examinations is usually provided in the form of the first and second reagents (solutions), which are sequentially mixed with the test sample in use. One or both of (a) and (b) in each of the forms A to D can be included in the first or second reagents. The methods of including (a) and (b) may be appropriately selected depending on the measuring device for the clinical examination and the design of the assay reagent (such as capability and usability). Although, preferably, both (a) and (b) of the form A are included in the second reagent, (a) and (b) of the form A may also be included in the first and second reagents, respectively, in a preferred manner.

Although the representative forms of the assay and assay reagent of the present invention have been described as an example, it can obviously be understood that the present invention can be implemented in various forms well known to those skilled in the art, such as competitive immunoassay, on the condition that the antibody of the present invention is used.

In the assay and assay reagent of the present invention described above, the cross-reactivity with porcine insulin is less than 18 %. The required level of the cross-reactivity varies depending on a purpose of assay of human insulin and is preferably less than 15 %, more preferably less than 10 %, further preferably less than 5 % to less than 2 %, particularly preferably less than 1 %. If the antibody of the present invention is used, the substantial cross-reactivity can be evaluated as 0 % and, therefore, the assay and assay reagent can be designed with cross-reactivity less than 1 % such as 0.9 % to 0.01 %.

Quantitative evaluating methods of the cross-reactivity in the assay and assay reagent include using the assay and assay reagent desired for the evaluation of the cross-reactivity to (1) obtain $IC_{50}$ (50 % inhibition concentration) by performing a competitive test with a test compound, (2) obtain a rate to a theoretical concentration by measuring a concentration of a test compound, or (3) obtain an arithmetic mean (average cross-reactivity) by measuring serial dilution samples of a test compound and obtaining a cross-reactivity of each sample in (2). A specific calculation formula of (2) can be as follows:

$$\text{cross-reactivity (rate) (\%)} = \text{measured concentration of test compound/theoretical concentration of test compound} \times 100.$$

Although the comparison should be made in terms of mole in the strict sense of the cross-reactivity, molecular weights of cross-reactive materials such as human insulin and porcine insulin of the present invention are the same or similar and, therefore, evaluations can be made by calculation performed simply in terms of mass without mole conversion.

The present invention also provides a method of measuring exogenous insulin such as insulin analogs and insulin derived from animal species other than human administered for treatment and the method includes the following steps. A concentration of exogenous insulin administered for treatment can be obtained by obtaining a human insulin concentration by the human insulin assay in combination with a step of measuring human insulin and exogenous insulin in order to obtain a total concentration of the human insulin and exogenous insulin, and subtracting the human insulin concentration from the total concentration.

"Samples" to be detected in an assay using the antibody of the present invention can mainly be body fluids (biological samples) derived from a living body (organism) and are not particularly limited as long as the samples contain human insulin. The samples can preferably include blood, serum, plasma, urine, saliva, phlegm, pancreas extract, etc., more preferably, blood, serum, and plasma.

Although the present invention will be described in more detail with reference to examples, the present invention is not limited to these examples.

EXAMPLES

[Test Example 1] Method of Producing Monoclonal Antibody of the Present Invention

1. Preparation of Immunizing Antigen

After human insulin (recombinant; Fitzgerald Industries International, 30-AI51) was mixed 1:1 with complete Freund's adjuvant (Wako Pure Chemical Industries, Ltd.), connected syringes were used for producing emulsion to be used as the immunizing antigen.

2. Production of Hybridoma

The immunizing antigen was subcutaneously injected into the dorsal regions of female BALB/c mice (20 to 50 μg per mouse). This operation (immunization) was repeated twice per week. After three weeks from the start of immunization, antiserum was acquired from the blood sample, spleen was extracted from the mouse having a high antibody titer for the antiserum in a test with solid-phase antigen ELISA described later, and cell fusion was performed by a routine procedure using 50% PEG 1450 (Sigma). SP2/O myeloma cells were used. The acquired fused cells were suspended in RPMI 1640 media that contained HAT (hypoxanthine, aminopterin, thymidine), 15% fetal bovine serum, and 10% BM-Condimed H1 Hybridoma Cloning Supplement (Roche Diagnostics K.K.) at $2.5\times10^6$ cells/mL in terms of spleen cells and were dispensed in a 96-well culture plate in 0.2-mL aliquots. The fused cells were cultivated at 37 °C in a 5% $CO_2$ incubator.

3. Screening of Hybridoma Producing the Monoclonal Antibody of the Present Invention

After seven days from the cell fusion, the culture supernatant was used for performing solid-phase antigen ELISA described later as primary screening to select wells that exhibited a high reactivity to human insulin as primary positive wells. The cells in the primary positive wells were serially passaged in a 24-well plate. After two days of serial cultivation, the culture supernatant was used to perform competitive ELISA of human insulin described later as secondary screening to select wells that exhibited a high reactivity to human insulin as secondary positive wells. A reactivity assay using Biacore (registered trademark) was performed as tertiary screening to select wells having a specific reactivity only to human insulin and having no cross-reactivity to proinsulin, insulin analogs (insulin lispro, insulin aspart, insulin glargine, insulin detemir, and insulin glulisine), porcine insulin, and bovine insulin as tertiary positive wells. For quaternary screening, the cells in the tertiary positive wells were cultivated and the culture supernatant was used to perform competitive ELISA between human insulin and a peptide fragment consisting of the sequence "RGFFYTPKT" of the C-terminal region of the human insulin β-chain to select wells having no reactivity to the peptide fragment and having high reactivity to human insulin as quaternary positive wells (the peptide fragment consists of the amino acid sequence indicated by SEQ ID NO. 1; the amino acid sequence of the peptide is different from porcine insulin only in that the C-terminal amino acid is "T" (the C-terminal amino acid of porcine insulin is "A")).

3-1. Production of the Solid-Phase Antigen ELISA Plate

Human Insulin (Fitzgerald Industries International, 30-AI51) prepared at a concentration of 1 μg/mL with 10 mM phosphate-buffered saline (PBS) (pH 7.2) containing 150 mM sodium chloride was solid-phased as a screening antigen on a 96-well plate at 50 μL/well and was allowed to stand overnight at 4°C. After washing three times with 400 μL/well of PBS solution containing 0.05% Tween (registered trademark) 20 and 0.1% ProClin 300 (Supelco; PBST), PBST containing 1% BSA (BSA-PBST) was dispensed at 100 μL/well and allowed to stand one hour at room temperature for blocking to produce a solid-phase antigen ELISA plate. The solid-phase antigen ELISA plate was washed three times with PBST and used for solid-phase antigen ELISA as well as tests described in the test examples and the examples. Human insulin used in the test examples and the examples described herein is converted into the international unit by 26 IU/mg.

3-2. Solid-Phase Antigen ELISA

(i) Mouse antiserum acquired from blood samples diluted stepwise with BSA-PBST or culture supernatant of the fused cells was dispensed on the solid-phase antigen ELISA plate at 50 μL/well and allowed to stand one hour at room temperature.
(ii) After washing three times with PBST, a solution of HRP-Gt F(ab')$_2$-Anti-Mouse Ig's (BioSource, AMI4404) diluted 5000 times with BSA-PBST was dispensed at 50 μL/well and allowed to stand one hour at room temperature.
(iii) After washing three times with PBST, OPD (Tokyo Chemical Industry Co., Ltd.) was dissolved at 2 mg/mL in 0.2 M citrate buffer solution containing 0.02% hydrogen peroxide/water (hereinafter, substrate-dissolving solution), added at 50 μL/well, and allowed to stand one hour at room temperature.
(iv) Furthermore, 1.5 N sulfuric acid containing 1 mM EDTA (hereinafter, reaction stop liquid) was added at 50 μL/well, and absorbance was measured at a wavelength of 492 nm using Titertek (registered trademark) Multiskan Plus MK II (Flow Laboratories Inc).

3-3. Competitive ELISA of Human Insulin

(i) Solutions of human insulin (Fitzgerald Industries International, 30-AI51) diluted with BSA-PBST at 0, 2.5, 5, and 10 μg/mL were dispensed on a solid-phase antigen ELISA plate at 25 μL/well.
(ii) Culture supernatant of the fused cells diluted to 5 and 25 times with BSA-PBST or undiluted solution of the culture supernatant was then dispensed at 25 μL/well and allowed to stand one hour at room temperature.
(iii) The subsequent operations were performed in the same manner as steps (ii) to (iv) of "3-2. Solid-Phase Antigen ELISA" described above.

3-4. Reactivity Assay between Antibody and Test Compounds Using Biacore (Registered Trademark)

Biacore (registered trademark) T100 (GE healthcare, JJ-1037-02) was used to perform a screening test of a hybridoma, using reaction specificity of an antibody as an index.

(i) Mouse Antibody Capture Kit (GE Healthcare, BR-1008-38) and Amine Coupling Kit (GE Healthcare, BR-1000-50) are used to immobilize Anti-Mouse IgG antibodies to Sensor Chip CM5 (GE Healthcare, BR-1005-30).
(ii) Undiluted solution of the culture supernatant of the fused cells was added for 300 seconds at a flow rate of 30 $\mu$L/min to Sensor Chip CM5 with Anti-Mouse IgG antibodies immobilized so as to capture antibodies contained in the culture supernatant with Anti-Mouse IgG antibodies.
(iii) HBS-EP+ 10$\times$ (running buffer) (GE Healthcare, BR-1006-69) was adjusted to pH 8.5 with NaOH and then finally diluted 10 times with purified water to prepare an HBS-EP+ working solution, which was used for diluting the following test compounds to 10 ng/mL. The diluted solutions of the test compounds were added at two concentrations 0 ng/mL and 10 ng/mL for 120 seconds each at a flow rate of 30 $\mu$L/min to Sensor Chip CM5 with Anti-Mouse IgG antibodies immobilized. A time for free-running dissociation was set to 120 seconds in this case. The formulation of the HBS-EP+ working solution consists of 0.01 M HEPES (pH 8.5), 0.15 M sodium chloride, 3 mM EDTA, and 0.005 % Surfactant P20.

<Test Compounds>

(1) human insulin: Fitzgerald Industries International, 30-AI51
(2) proinsulin: IRR, Proinsulin, Human, for Immunoassay; NIBSC code: 84/611
(3) insulin analogs
insulin lispro, 100 units/mL: Eli Lilly Japan K.K.
insulin aspart, 100 units/mL: Novo Nordisk Pharma Ltd.
insulin glargine, 100 units/mL: sanofi-aventis K.K.
insulin detemir, 100 units/mL: Novo Nordisk Pharma Ltd.
insulin glulisine, 100 units/mL: sanofi-aventis K.K.
(4) insulin derived from animal species other than human
bovine insulin: SIGMA I5500
porcine insulin: WAKO 091-04211

(iv) Glycine 1.5 (GE Healthcare, BR-1003-54) and Glycine 2.0 (GE Healthcare, BR-1003-55) were mixed 1:1 to form regenerating solution, and regenerating treatment was performed for 180 seconds.

3-5. Competitive ELISA of Synthetic Peptide Fragment

(i) A peptide fragment consisting of the sequence "RGFFYTPKT" (SEQ ID NO. 1) of the C-terminal region of the human insulin $\beta$-chain was produced. The peptide fragment was produced by a peptide automatic synthesizer and was synthesized and purified in accordance with the Fmoc method. HPLC was used to confirm that the purity of the peptide was equal to or greater than 95 %. A mass spectroscope (MALDI-TOF) was used to confirm that the molecular weight was the same as the theoretical value.
(ii) The synthetic peptide fragment produced at (i) or human insulin (Fitzgerald Industries International, 30-AI51) diluted with BSA-PBST at 0, 2.5, 5, and 10 $\mu$g/mL were dispensed on a solid-phase antigen ELISA plate at 25 $\mu$L/well.
(iii) The subsequent operations were performed in the same manner as steps (ii) and (iii) of "3-3. Competitive ELISA of Human Insulin" described above.

4. Screening of Hybridoma Producing Monoclonal Antibody A Used in Combination with the Monoclonal Antibody of the Present Invention

After seven days from the cell fusion, the culture supernatant was used for performing solid-phase antigen ELISA as primary screening to select wells that exhibited a high reactivity to human insulin as primary positive wells.

The cells in the primary positive wells were serially passaged in a 24-well plate. After two days of serial cultivation, the culture supernatant was used to perform competitive ELISA as secondary screening to select wells that exhibited a high reactivity to human insulin as secondary positive wells.

5. Cloning and Monoclonal Antibody Collection

Hybridomas selected by the screenings of 3. (after the completion of the quaternary screening) and 4. (after the completion of the secondary screening) described above were cloned by a limiting dilution method to acquire hybridomas 66224 and 66408, respectively. To collect the monoclonal antibodies produced by the hybridomas, the hybridomas were intraperitoneally administered, in an amount corresponding to $0.5 \times 10^6$ cells, to a 12-week-old female BALB/c mouse intraperitoneally injected with 0.5 mL of pristane two weeks before the administration of the hybridomas. The ascites were collected after 14 days, and the supernatants were acquired by centrifugation. The supernatants were mixed with the same amount of adsorption buffer solution (3 mol/L NaCl, 1.5 mol/L Glycine-NaOH buffer solution, pH 8.5) and then filtrated. The filtrates were passed through a protein A sepharose column equilibrated with adsorption buffer solution to adsorb the antibodies in the filtrates using the column, and the antibodies were eluted with 0.1 mol/L citrate buffer solution (pH 3.0). After neutralizing the eluate with 1 mol/L Tris-HCl buffer solution (pH 8.0), dialysis was performed with PBS to collect the antibodies.

The antibodies, referred to as the 66224-antibody and 66408-antibody, were subsequently used in tests.

Hybridomas producing the 66224-antibody and 66408-antibody were deposited at International Patent Organism Depositary, National Institute of Advanced Industrial Science and Technology (Address: Tsukuba Central 6, 1-1-1 Higashi, Tsukuba, Ibaraki, Japan) on 26 June 2009 under the accession numbers FERM BP-11314 and FERM BP-11315, respectively.

[Test Example 2] Cross-Reactivity of Monoclonal Antibody of the Present Invention with Human Insulin, Proinsulin, Insulin Analogs, Porcine Insulin, and Bovine Insulin

A test was performed using Biacore (registered trademark) T100 for cross-reactivity of the 66224-antibody or the 66408-antibody with proinsulin, insulin analogs, porcine insulin, and bovine insulin. The method of the test was the same as 3-4 of the first example and the test was performed for the 66224-antibody to confirm the specific reactivity to the test compounds after purification of the antibody and was also performed for the 66408-antibody to confirm the specific reactivity to the test compounds after purification of the antibody.

1. Test Method

The 66224-antibody or the 66408-antibody was captured by Anti-Mouse IgG antibodies immobilized on Sensor Chip CM5, and insulin, proinsulin, various insulin analogs, porcine insulin, and bovine insulin were added as the test compounds to evaluate the reactivity. The specific operational procedure is as follows and the test compounds are the same as the first test example.

(i) Anti-Mouse IgG antibodies were immobilized on Sensor Chip CM5.
(ii) The 66224-antibody or 66408-antibody was diluted with the HBS-EP+ working solution (pH 8.5) to 5 $\mu$g/mL and added at a flow rate of 30 $\mu$L/min for 300 seconds so as to allow Sensor Chip CM5 immobilizing Anti-Mouse IgG antibodies to capture the 66224-antibody or 66408-antibody.
(iii) The test compounds diluted with the HBS-EP+ working solution (pH 8.5) were added at two concentrations 0 ng/mL or 10ng/mL for 120 seconds at a flow rate of 30 $\mu$L/min to Sensor Chip CM5 with Anti-Mouse IgG antibodies immobilized. A time for free-running dissociation was set to 120 seconds in this case.
(iv) Glycine 1.5 and Glycine 2.0 were mixed 1:1 to form regenerating solution, and regenerating treatment was performed for 180 seconds.

2. Results

2-1. Reactivity of the 66224-Antibody

For the 66224-antibody, Biacore (registered trademark) T100 was used to confirm reactivity with human insulin, proinsulin, various insulin analogs (insulin lispro, insulin aspart, insulin glargine, insulin detemir, and insulin glulisine), porcine insulin, and bovine insulin. The results are depicted in Fig. 2. In Fig. 2, the vertical axis represents a mass change due to reaction (binding of an antigen to an antibody) on the sensor surface (Response), and "RU" represents a unit unique to the Biacore (registered trademark) assay system. The horizontal axis represents time (Time) in "seconds (s)" (the same applies below). While a reactivity of 2.0 RU was detected at a human insulin concentration of 10 ng/mL, RU was calculated as zero for the other test compounds (10 ng/mL) and no reactivity was detected (Fig. 2). Therefore, it is confirmed that the 66224-antibody is an "antibody reactive with human insulin and nonreactive with porcine insulin" as well as an "antibody nonreactive with one or more of bovine insulin, proinsulin, and insulin analogs" in this description.

2-2. Reactivity of the 66408-Antibody

For the 66408-antibody, Biacore (registered trademark) T100 was used to confirm reactivity with proinsulin, various insulin analogs (insulin lispro, insulin aspart, insulin glargine, and insulin detemir), porcine insulin, and bovine insulin. The results are depicted in Fig. 3. While a reactivity of 2.5 RU was detected at a human insulin concentration of 10 ng/mL, RU of bovine insulin was calculated as zero and no reactivity was detected to bovine insulin. On the other hand, RU was calculated as 0.6 to 13 for the other test compounds and the reactivity was detected (Fig. 3). Therefore, it is confirmed that the 66408-antibody is an "antibody A at least reactive with human insulin" (antibody reactive with human insulin and reactive with (a subset of) insulin derived from animal species other than human and insulin analogs) in this description.

[Test Example 3] Confirmation of Recognized Epitope of the 66224-Antibody

As a result of the second test example, it is confirmed that the 66224-antibody is reactive with human insulin and nonreactive with porcine insulin. Human insulin and porcine insulin are different from each other only in that the β-chain C-terminal amino acid is "T" or "A" (Fig. 1) and it was believed that the 66224-antibody identifies and recognizes human insulin and porcine insulin from a difference in the one amino acid. Therefore, to confirm the recognized epitope of the 66224-antibody, competitive ELISA was performed using a synthetic peptide fragment (produced at 3-5 of [test example 1] described above) including the amino acid sequence site different between human insulin and porcine insulin. If the 66224-antibody reacts (competes) with the synthetic peptide fragment in this test, it is believed that the 66224-antibody recognizes the difference (substitution) in the primary structure of the amino acid sequence including the insulin β-chain C-terminal. If the 66224-antibody does not react with the synthetic peptide fragment, it can be believed that the 66224-antibody recognizes the conformation formed by the β-chain C-terminal amino acid sequence region in the human insulin molecule.

1. Test Method

The presence of reactivity between the synthetic peptide and the 66224-antibody was checked in accordance with the following procedure.

(i) Human insulin (Fitzgerald Industries International, 30-AI51) was diluted using PBS to 1 μg/mL, added to a 96-well plate at 50 μL per well, and allowed to stand two hours at room temperature.
(ii) Washing was performed three times with 400 μlL/well of PBS solution containing 0.05% Tween (registered trademark) 20 and 0.1% ProClin 300 (Supelco) (PBST).
(iii) BSA-PBST was added at 100 μL per well and allowed to stand one hour at room temperature.
(iv) Added BSA-PBST solution was completely removed by aspiration.
(v) As a competitive test compound, human insulin or the synthetic peptide fragment was diluted with BSA-PBST at 0, 2.5, 5, and 10 μg/mL and added at 25 μL per well, and the 66224-antibody is diluted to 2 μg/mL with BSA-PBST, added thereto at 25 μL per well, and allowed to stand one hour at room temperature.
(vi) Washing was performed three times with 400 μL/well of the PBST solution.
(vii) HRP-labeled goat anti-mouse IgGy (SouthernBiotech, 1030-05) was diluted 5000 times, added at 50 μL per well, and allowed to stand one hour at room temperature.
(viii) Washing was performed three times with 400 μL/well of the PBST solution.
(ix) OPD (Tokyo Chemical Industry Co., Ltd.) was dissolved at 2 mg/mL in the substrate-dissolving solution, added to each well at 50 μL, and allowed to stand one hour at room temperature.
(x) The reaction stop liquid was added at 50 μL per well, and absorbance was measured at a wavelength of 492 nm using Titertek (registered trademark) Multiskan Plus MK II (Flow Laboratories Inc).

The diluting solution was BSA-PBST unless otherwise stated.

2. Results

Test results are depicted in Table 1 and Fig. 4.

Since the 66224-antibody exhibited reactivity with human insulin, when human insulin was used as the test compound competitive with the solid-phased human insulin, the reactivity was reduced depending on the concentration. This is because an amount of the 66224-antibody reactive with the solid-phased human insulin is reduced since the 66224-antibody is absorbed by human insulin in the competitive solution. However, when the synthetic peptide fragment was used as the competitive test compound, no variation in the reactivity was observed depending on the concentration of the synthetic peptide fragment. Therefore, it is confirmed that the 66224-antibody has no reactivity with the synthetic peptide fragment. From the above results, it is believed that the 66224-antibody has no reactivity with the primary structure of the amino acid sequence including the amino acid of the human insulin β-chain C-terminal and recognizes the conformation formed by the β-chain C-terminal amino acid sequence region in the human insulin molecule.

[Table 1]

| Antigen concentration (μg/mL) \ Antigen type | Absorbance (Abs) | |
| --- | --- | --- |
| | Human insulin | Peptide fragment |
| 0 | 0.786 | 0.780 |
| 2.5 | 0.303 | 0.813 |
| 5 | 0.208 | 0.834 |
| 10 | 0.154 | 0.816 |

[Example 1] Assay of Human Insulin Using Combination of Monoclonal Antibody of the Present Invention and Antibody A at least Reactive With Human Insulin: 1

<LTIA>

1. Production of Latex Particles

A glass reaction container (capacity: 2 L) equipped with a stirring machine, reflux condenser, thermal sensing device, nitrogen introduction tube, and jacket was filled with 1100 g of distilled water, 200 g of styrene, 0.2 g of sodium styrene sulfonate, and aqueous solution of 1.5 g of potassium persulfate dissolved in 50 g of distilled water, and after the inside of the container was replaced with nitrogen gas, polymerization was performed for 48 hours while stirring at 70 °C. After the end of polymerization, the solution was filtrated with a filter paper to extract latex particles. A transmission electron microscope (JEOL Ltd., model "JEM-1010") was used for imaging the latex particles at a magnification of 10000 times and analyzing diameters of at least 100 acquired latex particles to determine the average particle diameter. The obtained average particle diameter was 0.3 μm.

2. Preparation of the Anti-Insulin Antibody-Sensitized Latex Particle

2-1. Production of 66224-Antibody-Sensitized Latex Particle Solution

To 1.0% latex solution having an average particle diameter of 0.3 μm [in 5 mM Tris-HCl buffer solution (hereinafter, Tris-HCl), pH 8.5], the same volume of 66224-antibody solution, diluted to 0.60 mg/mL with 5 mM Tris-HCl (pH 8.5), was added and stirred at 4 °C for two hours. The same volume of 5 mM Tris-HCl (pH 8.5) containing 0.5% BSA was subsequently added to the mixed solution of the latex and the antibody above and stirred at 4°C for one hour. After the solution was centrifuged and supernatant removed, the precipitate was resuspended in 5 mM Tris-HCl (pH 8.5) to produce a 66224-antibody-sensitized latex particle solution.

2-2. Production of 66408-Antibody-Sensitized Latex Particle Solution

The latex having an average particle diameter of 0.3 μm was used for producing a 66408-antibody-sensitized latex particle solution in the same manner as above.

3. Preparation of Reagents

3-1. Preparation of First Reagent

Five (5) millimolar Tris-HCl (pH 8.5) containing 500 mM of sodium chloride and 0.2% BSA was prepared as the first reagent.

3-2. Preparation of Second Reagent

The same volumes of the 66224-antibody- and 66408-antibody-sensitized latex particle solutions were mixed and diluted with 5 mM Tris-HCl (pH 8.5) such that absorbance of 5.0 Abs was achieved at a wavelength of 600 nm to prepare the second reagent.

4. Assay

The first and second reagents were combined, and human insulin concentration-dependent formation of particle aggregate was identified using a Hitachi 7170 Automated Analyzer. In particular, 150 μL of the first reagent was added to 10 μL of human insulin solutions at concentrations of 0, 5, 25, 50, 100, and 200 μU/mL and heated at 37 °C for 5 minutes. Subsequently, 50 μL of the second reagent was added, followed by stirring. After five minutes, changes in absorbance associated with agglutination formation were measured at main wavelength of 570 nm and sub-wavelength of 800 nm.

5. Assay Result

The assay result is depicted in Fig. 2. From Table 2, it is confirmed that the signal increases depending on the human insulin concentration and can be quantitated.

[Table 2]

| Human insulin concentration (μU/mL) | Absorbance (mAbs) |
|---|---|
| 0 | 15.1 |
| 5 | 17.2 |
| 25 | 24.5 |
| 50 | 34.6 |
| 100 | 118.8 |
| 200 | 184.1 |

[Example 2] Assay of Human Insulin Using Combination of Monoclonal Antibodies of the Present Invention: 2

<Sandwich ELISA>

Either of the 66224-antibody or the 66408-antibody was solid-phased (primary antibody) and combined with the rest as a labeled antibody (secondary antibody). Sandwich ELISA was used to test the reactivity with human insulin, proinsulin, insulin analogs, rabbit insulin, canine insulin, porcine insulin, and bovine insulin.

1. Antibodies and Test Compounds used

(1) Monoclonal Antibodies

66224-antibody: 4.03 mg/mL
66408-antibody: 9.04 mg/mL

(2) Test Compounds

Human insulin, proinsulin, various insulin analogs (insulin lispro, insulin aspart, insulin glargine, insulin detemir, and insulin glulisine), porcine insulin, and bovine insulin used were the same as the first and second test examples. Rabbit insulin and canine insulin are as follows:

rabbit insulin: Morinaga Institute of Biological Science, Inc., 200723; and
canine insulin: Morinaga Institute of Biological Science, Inc., 200722.

2. Sandwich ELISA assay

(i) The solution of the 66224-antibody or 66408-antibody diluted to 2 $\mu$g/mL with PBS was solid-phased in a 96-well plate at 50 $\mu$U/well and allowed to stand two hours at room temperature.
(ii) After washing three times with 400 $\mu$U/well of PBST, BSA-PBST was dispensed at 100 $\mu$L/well and allowed to stand one hour at room temperature for blocking in order to produce a sandwich ELISA plate.
(iii) The solution of each of human insulin, proinsulin, various insulin analogs, porcine insulin, bovine insulin, rabbit insulin, and canine insulin diluted with BSA-PBST to 0, 2.5, 5, and 10 ng/mL was dispensed on the sandwich ELISA plate at 50 $\mu$L/well and allowed to stand one hour at room temperature.
(iv) After washing three times with PBST, a solution of a biotin-labeled 66224-antibody or 66408-antibody diluted to 1 $\mu$g/mL with BSA-PBST was dispensed at 50 $\mu$L/well and allowed to stand one hour at room temperature.
(v) After washing three times with PBST, a solution of Immuno Pure (registered trademark) Streptavidin, HRP-Conjugated (PIERCE, Prod# 21126) diluted 5000 times with BSA-PBST was dispensed at 50 $\mu$L/well and allowed to stand one hour at room temperature.
(vi) After washing three times with PBST, OPD (Tokyo Chemical Industry) was dissolved at 2 mg/mL in the substrate-dissolving solution, added at 50 $\mu$L/well, and allowed to stand one hour at room temperature.
(vii) The reaction stop solution was added at 50 $\mu$L/well, and absorbance was measured at 492 nm using Titertek (registered trademark) Multiskan Plus MK II (Flow Laboratories).

3. Results

3-1. 66224 Solid-Phase Antibody Plate Assay Results

Test results are depicted in Table 3 and Fig. 5.
When the 66224-antibody was used as the primary antibody and the 66408-antibody was used as the secondary antibody, a concentration-dependent increase in absorbance was observed for human insulin, while no concentration-dependent increase in absorbance was observed for the other test compounds and the measured absorbance was limited to the extent of measurement error.

[Table 3]

| Primary antibody | 66224-antibody | | | | |
|---|---|---|---|---|---|
| Secondary antibody | Biotin–66408-antibody | | | | |
| Antigen concentration \ Antigen type | Human insulin | Proinsulin | Insulin lispro | Insulin aspart | Insulin glargine |
| 0 n g／m L | 0. 000 | 0. 000 | 0. 000 | 0. 000 | 0. 000 |
| 2. 5 n g／m L | 0. 076 | −0. 004 | 0. 004 | 0. 007 | 0. 013 |
| 5 n g／m L | 0. 180 | −0. 003 | 0. 002 | 0. 006 | 0. 006 |
| 1 0 n g／m L | 0. 693 | 0. 002 | 0. 004 | 0. 009 | 0. 016 |

Absorbance（A b s）

| Primary antibody | 66224-antibody | | | | |
|---|---|---|---|---|---|
| Secondary antibody | Biotin–66408-antibody | | | | |
| Antigen concentration \ Antigen type | Insulin detemir | Insulin glulisine | Porcine insulin | Bovine insulin | Rabbit insulin | Canine insulin |
| 0 n g／m L | 0. 000 | 0. 000 | 0. 000 | 0. 000 | 0. 000 | 0. 000 |
| 2. 5 n g／m L | 0. 001 | −0. 002 | −0. 002 | 0. 003 | −0. 006 | 0. 001 |
| 5 n g／m L | 0. 007 | −0. 006 | −0. 001 | −0. 005 | −0. 005 | −0. 003 |
| 1 0 n g／m L | 0. 005 | 0. 001 | 0. 012 | 0. 004 | −0. 004 | −0. 001 |

Absorbance（A b s）

3-2. 66408 Solid-Phase Antibody Plate Assay Results

Test results are depicted in Table 4 and Fig. 6.

When the 66408-antibody was used as the primary antibody and the 66224-antibody was used as the secondary antibody, a concentration-dependent increase in absorbance was observed for human insulin, while no concentration-dependent increase in absorbance was observed for the other test compounds and the measured absorbance was limited to the extent of measurement error.

[Table 4]

| Primary antibody | 66408-antibody | | | | |
|---|---|---|---|---|---|
| Secondary antibody | Biotin–66224-antibody | | | | |
| Antigen concentration \ Antigen type | Human insulin | Proinsulin | Insulin lispro | Insulin aspart | Insulin glargine |
| 0 n g／m L | 0. 000 | 0. 000 | 0. 000 | 0. 000 | 0. 000 |
| 2. 5 n g／m L | 0. 041 | 0. 003 | 0. 001 | 0. 003 | −0. 008 |
| 5 n g／m L | 0. 247 | −0. 003 | 0. 017 | −0. 001 | −0. 010 |
| 1 0 n g／m L | 0. 926 | 0. 004 | −0. 003 | 0. 006 | −0. 003 |

Absorbance（A b s）

| Primary antibody | 66408-antibody | | | | |
|---|---|---|---|---|---|
| Secondary antibody | Biotin–66224-antibody | | | | |
| Antigen concentration \ Antigen type | Insulin detemir | Insulin glulisine | Porcine insulin | Bovine insulin | Rabbit insulin | Canine insulin |
| 0 n g／m L | 0. 000 | 0. 000 | 0. 001 | 0. 000 | 0. 000 | 0. 000 |
| 2. 5 n g／m L | 0. 001 | 0. 002 | −0. 002 | 0. 001 | −0. 008 | −0. 013 |
| 5 n g／m L | −0. 004 | 0. 025 | 0. 000 | −0. 001 | −0. 005 | −0. 003 |
| 1 0 n g／m L | 0. 004 | 0. 015 | 0. 000 | 0. 003 | 0. 003 | −0. 001 |

Absorbance（A b s）

4. Discussion

From the result of Example 2, no test compound other than human insulin is detected regardless of whether the 66224- or 66408-antibody is used as the primary or secondary antibody and, therefore, it is understood that human insulin alone can be quantitated by the assay of the present invention without being affected by the test compounds. In other words, with the assay of the present invention, human insulin only could specifically be assayed without being affected by proinsulin, insulin analogs, porcine insulin, bovine insulin, rabbit insulin, and canine insulin. Based on the result of Example 2, a human insulin assay and an assay reagent that have extremely low cross-reactivity to test compounds other than human insulin compared to commercial reagents (e.g., cross-reactivity less than 18 % to porcine insulin) or those that have the cross-reactivity of substantially zero percent can be formed.

In the result of the cross-reactivity test of the monoclonal antibody of the present invention using Biacore (registered trademark) T100 of the second test example, the 66224-antibody was reactive with human insulin and nonreactive with any other test compounds. On the other hand, since the 66408-antibody had reactivity with all the test compounds other than bovine insulin, it is believed that higher specificity of the 66224-antibody to human insulin enables the human-insulin-specific assay.

From the third test example, it is believed that the 66224-antibody recognizes the conformation of human insulin involved with the amino acid sequence of the β-chain C-terminal region of human insulin and, therefore, the human-insulin-specific assay is enabled by the property of using a certain sterically-different site of human insulin and porcine insulin as an epitope, which is one feature of the present invention.

INDUSTRIAL AVAILABILITY

With the present invention, human insulin can accurately be assayed without being affected by insulin derived from animal species other than human such as porcine insulin, proinsulin, and insulin analogs. In particular, since only endogenous human insulin secreted from the beta cells of a diabetic patient can accurately be assayed by the present invention even in the case of a diabetic patient subjected to the administration of insulin analogs etc., a clinical condition of the diabetic patient can accurately be understood.

By combining an assay result of the assay of the present invention with, for example, an assay result from a conventional insulin assay showing cross-reactivity to insulin derived from animal species other than human and insulin analogs in addition to human insulin, endogenous insulin produced by a diabetic patient can be distinguished from and assayed along with the insulin analogs and insulin derived from animal species other than human; the contribution of administered exogenous insulin to medical treatment can be understood; and, therefore, the present invention is very useful.

ACCESSION NUMBER

**[0112]**

    (1) FERM BP-11314
    (2) FERM BP-11315
    (3) FERM BP-11233
    (4) FERM BP-11234

[Reference to Deposited Biological Material]

(1) Hybridoma 66224 producing the 66224-antibody

i) Name and address of depository institution at which the biological materials were deposited.

    International Patent Organism Depositary, National Institute of Advanced Industrial Science and Technology
    Tsukuba Central 6, 1-1-1 Higashi, Tsukuba, Ibaraki 305-8566, Japan

ii) Date of biological material deposit in the depository institution in i).

26 June 2009 (original deposit date)
6 December 2010 (date of transfer to the Budapest Treaty from the original deposit)

iii) Accession number for the deposition assigned by the depository institution in i). FERM BP-11314

(2) Hybridoma 66408 producing the 66408-antibody

i) Name and address of depository institution at which the biological materials were deposited.

Same as (1)

ii) Date of biological material deposit in the depository institution in i).

26 June 2009 (original deposit date)
6 December 2010 (date of transfer to the Budapest Treaty from the original deposit)

iii) Accession number for the deposition assigned by the depository institution in i).

FERM BP-11315

(3) Hybridoma 66221 producing the 66221-antibody

i) Name and address of depository institution at which the biological materials were deposited.

Same as (1)

ii) Date of biological material deposit in the depository institution in i).

8 April 2009 (original deposit date)
17 February 2010 (date of transfer to the Budapest Treaty from the original deposit)

iii) Accession number for the deposition assigned by the depository institution in i).

FERM BP-11233

(4) Hybridoma 66226 producing the 66226-antibody

i) Name and address of depository institution at which the biological materials were deposited.

Same as (1)

ii) Date of biological material deposit in the depository institution in i).

8 April 2009 (original deposit date)
17 February 2010 (date of transfer to the Budapest Treaty from the original deposit)

iii) Accession number for the deposition assigned by the depository institution in i).

FERM BP-11234

<110> SEKISUI MEDICAL Co., LTD

<120> Insulin Assay and Insulin Assay reagent

<130> 09P01268

<150> JP 2009-295260
<151> 2009-12-25

<160> 1

<170> PatentIn version 3.1

<210> 1
<211> 9
<212> PRT
<213> Artificial sequence

<220>
<223> peptide

<400> 1

        Arg Gly Phe Phe Tyr Thr Pro Lys Thr
        1               5

## Claims

1. A human insulin assay comprising the steps of:

   i. bringing an anti-human insulin antibody having the following properties (a) to (g) into contact with a biological sample; and
   ii. detecting the complex of the anti-human insulin antibody and human insulin formed during the contact step

      a) the antibody reacts with human insulin,
      b) the antibody does not react with porcine insulin
      c) the antibody does not react with bovine insulin,
      d) the antibody does not react with canine insulin,
      e) the antibody does not react with rabbit insulin,
      f) the antibody does not react with proinsulin, and
      g) the antibody does not react with an insulin analog,

   wherein the insulin analog is selected from a group consisting of insulin lispro, insulin aspart, insulin glargine, insulin detemir, and insulin glulisine,
   wherein

      h) the antibody does not react with a peptide fragment consisting of sequence RGFFYTPKT (SEQ ID NO. 1) and

      (i) the antibody recognizes a conformation of a β-chain C-terminal RGFFYTPKT region in a human insulin molecule.

2. The assay according to claim 1, wherein the conformation of the (3-chain C-terminal RGFFYTPKT region in a human insulin molecule is a conformation achievable in the following solution:

0.01 M HEPES (pH 8.5), 0.15 M sodium chloride, 3 mM EDTA, and 0.005 % Surfactant P20.

3. The assay according to any one of claims 1 to 2, wherein the anti-human insulin antibody is a monoclonal antibody.

4. The assay according to claim 3, wherein the anti-human insulin antibody is produced by a hybridoma of the accession number FERM BP-11314,
or
wherein the anti-human insulin antibody is capable of recognizing an epitope identical to an epitope recognized by a monoclonal antibody produced by the hybridoma of the accession number FERM BP-11314.

5. The human insulin assay according to any one of claims 1 to claim 4, wherein the anti-human insulin antibody is labeled with a detectable labeling material.

6. The assay according to any one of claims 1 to 5, further using:

an antibody A having a property of reacting at least with human insulin,

or
further using:

an antibody B having a property of specifically recognizing the anti-human insulin antibody.

7. The human insulin assay according to claim 6, wherein the anti-human insulin antibody, the antibody A, and the antibody B are monoclonal antibodies,
or
wherein the anti-human insulin antibody is a monoclonal antibody, and wherein the antibody A and the antibody B are a polyclonal antibody.

8. The human insulin assay according to claim 6 or 7, wherein the anti-human insulin antibody and/or the antibody A and the antibody B are immobilized to a solid phase.

9. The assay according to claim 8, wherein the solid phase is latex, and wherein insulin is measured by a latex immunoagglutination assay.

10. The human insulin assay according to claim 9 , wherein the anti-human insulin antibody is immobilized to a solid phase, wherein the antibody A and the antibody B is labeled with a labeling material, and wherein insulin is measured by ELISA or immunochromatography.

11. An exogenous insulin assay comprising the steps of:

(1) obtaining a total concentration of human insulin and exogenous insulin;
(2) obtaining a human insulin concentration with the insulin assay of any one of claims1 to 12; and
(3) obtaining an exogenous insulin concentration by subtracting the concentration obtained at (2) from the concentration obtained at (1).

12. An anti-human insulin antibody, wherein the anti-human insulin antibody is capable of recognizing an epitope identical to an epitope recognized by a monoclonal antibody produced by the hybridoma of the accession number FERM BP-11314.

13. An insulin assay reagent, wherein the insulin assay reagent uses the antibody of claim 12.

14. A human insulin assay reagent using the following two antibodies:

1) the anti-human insulin antibody of claim 12, and
2) an antibody A having a property of reacting at least with human insulin,

or

using the following two antibodies:

1) the anti-human insulin antibody of claim 12, and
2) an antibody B having a property of specifically recognizing the antibody of 1).

**15.** The human insulin assay reagent of claim 14, wherein the both antibodies of 1) and 2) are monoclonal antibodies, or

wherein the antibody of 1) is a monoclonal antibody, and wherein the antibody of 2) is a polyclonal antibody.

**16.** The human insulin assay reagent of claim 14 or 15, wherein the antibody of 1) and/or the antibody of 2) are immobilized to a solid phase.

**17.** The human insulin assay reagent of claim 16, wherein the solid phase is latex, and wherein insulin is assayed by a latex immunoagglutination assay, or

wherein the antibody of 1) is immobilized to a solid phase, wherein the antibody of 2) is labeled with a labeling material, and wherein insulin is assayed by ELISA or immunochromatography.

**18.** An exogenous insulin assay kit including the following assay reagents:

(1) a reagent for measuring a total insulin concentration of human insulin and exogenous insulin, and
(2) the human insulin assay reagent of any one of claims 13 to 17.

**Patentansprüche**

**1.** Humaner Insulin-Assay, umfassend die Schritte:

i. Inkontaktbringen eines anti-humanen Insulin-Antikörpers, der die folgenden Eigenschaften (a) bis (g) aufweist, mit einer biologischen Probe; und
ii. Detektieren des während des Kontaktschrittes gebildeten Komplexes des anti-humanen Insulin-Antikörpers mit humanem Insulin

a) der Antikörper reagiert mit humanem Insulin,
b) der Antikörper reagiert nicht mit procinem Insulin,
c) der Antikörper reagiert nicht mit bovinem Insulin,
d) der Antikörper reagiert nicht mit caninem Insulin,
e) der Antikörper reagiert nicht mit Kaninchen-Insulin,
f) der Antikörper reagiert nicht mit Proinsulin und
g) der Antikörper reagiert nicht mit einem Insulin-Analogon,
wobei das Insulin-Analogon ausgewählt wird aus der Gruppe, bestehend aus Insulin-Lispro, Insulin-Aspart, Insulin-Glargin, Insulin-Detemir und Insulin-Glulisin,
wobei
h) der Antikörper mit einem Peptidfragment, bestehend aus Sequenz RGFFYTPKT (SEQ ID NO: 1), nicht reagiert und
(i) der Antikörper eine Konformation einer $\beta$-Ketten-C-terminalen RGFFYTPKT-Region in einem humanen Insulin-Molekül erkennt.

**2.** Assay gemäß Anspruch 1, wobei die Konformation der $\beta$-Ketten-C-terminalen RGFFYTPKT-Region in einem humanen Insulin-Molekül eine Konformation ist, die in der folgenden Lösung erzielbar ist:

0,01 M HEPES (pH 8,5), 0,15 M Natriumchlorid, 3 mM EDTA und 0,005 % Tensid P20.

**3.** Assay gemäß einem der Ansprüche 1 bis 2, wobei der anti-humane Insulin-Antikörper ein monoklonaler Antikörper ist.

**4.** Assay gemäß Anspruch 3, wobei der anti-humane Insulin-Antikörper durch ein Hybridom der Zugangsnummer FERM BP 11314 produziert wird

oder

wobei der anti-humane Insulin-Antikörper imstande ist, ein Epitop zu erkennen, das mit einem Epitop identisch ist, das durch einen monoklonalen Antikörper, der durch das Hybridom der Zugangsnummer FERM BP-11314 produziert wird, erkannt wird.

5. Humaner Insulin-Assay gemäß einem der Ansprüche 1 bis Anspruch 4, wobei der anti-humane Insulin-Antikörper mit einem detektierbaren Markierungsmaterial markiert ist.

6. Assay gemäß einem der Ansprüche 1 bis 5, der im übrigen:

einen Antikrper A mit ener Eigenschaft, dass dieser wenigstens mit humanem Insulin reagiert, verwendet oder

einen Antikörper B mit einer Eigenschaft, dass dieser spezifisch den anti-humanen Insulin-Antikörper erkennt, verwendet.

7. Humaner Insulin-Assay gemäß Anspruch 6, wobei der anti-humane Insulin-Antikörper, der Antikörper A und der Antikörper B monoklonale Antikörper sind oder

wobei der anti-humane Insulin-Antikörper ein monoklonaler Antikörper ist und wobei der Antikörper A und der Antikörper B polyklonale Antikörper sind.

8. Humaner Insulin-Assay gemäß Anspruch 6 oder 7, wobei der anti-humane Insulin-Antikörper und/oder der Antikörper B und der Antikörper B an einer Festphase immobilisiert sind.

9. Assay gemäß Anspruch 8, wobei die Festphase Latex ist und wobei das Insulin durch einen Latex-Immunagglutinations-Assay gemessen wird.

10. Humaner Insulin-Assay gemäß Anspruch 9, wobei der anti-humane Insulin-Antikörper an einer Festphase immobilisiert ist, wobei der Antikörper A und der Antikörper B mit einem Markierungsmaterial markiert sind und wobei das Insulin durch ELISA oder Immunochromatographie gemessen wird.

11. Exogener Insulin-Assay, umfassend die Schritte:

(1) Ermitteln einer Gesamtkonzentration an humanem Insulin und exogenem Insulin;
(2) Ermitteln einer humanen Insulin-Konzentration mit dem Insulin-Assay gemäß einem der Ansprüche 1 bis 12; und
(3) Ermitteln einer exoenen Insulin-Konzentration durch Subtrahieren der in (2) ermittelten Konzentration von der in (1) ermittelten Konzentration.

12. Anti-humaner Insulin-Antikörper, wobei der anti-humane Insulin-Antikörper imstande ist, ein Epitop zu erkennen, das identisch ist zu einem Epitop, das durch einen monoklonalen Antikörper, der durch das Hybridom der Zugangsnummer FERM BP-11314 produziert wird, erkannt wird.

13. Insulin-Assay-Reagenz, wobei das Insulin-Assag-Reagenz den Antikörper gemäß Anspruch 12 verwendet.

14. Humanes Insulin-Assay-Reagenz, das die folgenden zwei Antikröper verwendet:

1) den anti-humanen Insulin-Antikörper gemäß Anspruch 12 und
2) einen Ankörper A mit einer Eigenschaft, dass dieser wenigstens mit humanem Insulin regiert,

oder

das die folgenden zwei Antikörper verwendet:

1) den anti-humanen Insulin-Antikörper gemäß Anspruch 12 und
2) einen Antikörper B mit einer Eigenschaft, dass dieser spezifisch den Antikörper von 1) erkennt.

15. Humanes Insulin-Assay-Reagenz gemäß Anspruch 14, wobei die beiden Antikörper von 1) und 2) monoklonale Antikörper sind,

oder

wobei der Antikörper von 1) ein monoklonaler Antikörper ist und wobei der Antikörper von 2) ein polyklonaler Antikörper ist.

16. Humanes Insulin-Assay-Reagenz gemäß Anspruch 14 oder 15, wobei der Antrikörper von 1) und/oder der Antikörper von 2) an einer Festphase immobilisiert sind.

17. Humanes Insulin-Assay-Reagenz gemäß Anspruch 16, wobei die Festphase Latex ist und wobei Insulin durch ein Latex-Immunoagglutinationsassay bestimmt wird,
oder
wobei der Antikörper von 1) an einer Festphase immobilisiert ist, wobei der Antikörper von 2) mit einem Markierungsmaterial markiert ist und wobei Insulin durch ELISA oder Immunochromatographie bestimmt wird.

18. Exogenes Isulin-Assay-Kit, umfassend die folgenden Assay-Reagenzien:

(1) ein Reagenz zum Messen einer Gesamtinsulinkonzentration an humanem Insulin und exogenem Insulin und
(2) das humane Insulin-Assay-Reagenz gemäß einem der Ansprüche 13 bis 17.

## Revendications

1. Dosage d'insuline humaine comprenant les étapes de :

i. amenée d'un anticorps anti-insuline humaine ayant les propriétés suivantes (a) à (g) en contact avec un échantillon biologique; et
ii. détection du complexe de l'anticorps anti-insuline humaine et de l'insuline humaine formé pendant l'étape de contact

a) l'anticorps réagit avec l'insuline humaine,
b) l'anticorps ne réagit pas avec l'insuline porcine
c) l'anticorps ne réagit pas avec l'insuline bovine,
d) l'anticorps ne réagit pas avec l'insuline canine,
e) l'anticorps ne réagit pas avec l'insuline de lapin,
f) l'anticorps ne réagit pas avec la proinsuline, et
g) l'anticorps ne réagit pas avec un analogue d'insuline, dans lequel l'analogue d'insuline est sélectionné à partir d'un groupe consistant en insuline lispro, insuline asparte, insuline glargine, insuline détémir et insuline glulisine,
dans lequel
h) l'anticorps ne réagit pas avec un fragment de peptide consistant en une séquence RGFFYTPKT (SEQ ID n° 1), et
(i) l'anticorps reconnaît une conformation d'une région RGFFYTPKT à terminaison C chaîne $\beta$ dans une molécule d'insuline humaine.

2. Dosage selon la revendication 1, dans lequel la conformation de la région RGFFYTPKT à terminaison C chaîne $\beta$ dans une molécule d'insuline humaine est une conformation réalisable dans la solution suivante :

0,01 M HEPES (pH 8,5), 0,15 M chlorure de sodium, 3 mM EDTA et 0,005 % d'agent tensioactif P20.

3. Dosage selon l'une quelconque des revendications 1 à 2, dans lequel l'anticorps anti-insuline humaine est un anticorps monoclonal.

4. Dosage selon la revendication 3, dans lequel l'anticorps anti-insuline humaine est produit par un hybridome du numéro matricule FERM BP-11314,
ou
dans lequel l'anticorps anti-insuline humaine est susceptible de reconnaître un épitope identique à un épitope reconnu par un anticorps monoclonal produit par l'hybridome du numéro matricule FERM BP-11314.

5. Dosage d'insuline humaine selon l'une quelconque des revendications 1 à 4, dans lequel l'anticorps anti-insuline humaine est étiqueté avec une matière d'étiquetage détectable.

6. Dosage selon l'une quelconque des revendications 1 à 5, utilisant en outre :

un anticorps A ayant une propriété de réaction au moins avec de l'insuline humaine,
ou
utilisant en outre :

un anticorps B ayant d'une propriété de reconnaissance spécifique de l'anticorps anti-insuline humaine.

7. Dosage d'insuline humaine selon la revendication 6, dans lequel l'anticorps anti-insuline humaine, l'anticorps A et l'anticorps B sont des anticorps monoclonaux,
ou
dans lequel l'anticorps anti-insuline humaine est un anticorps monoclonal, et dans lequel l'anticorps A et l'anticorps B sont un anticorps polyclonal.

8. Dosage d'insuline humaine selon la revendication 6 ou 7, dans lequel l'anticorps anti-insuline humaine et/ou l'anticorps A et l'anticorps B sont immobilisés sur une phase solide.

9. Dosage selon la revendication 8, dans lequel la phase solide est du latex, et dans lequel l'insuline est mesurée par un dosage d'immuno-agglutination de latex.

10. Dosage d'insuline humaine selon la revendication 9, dans lequel l'anticorps anti-insuline humaine est immobilisé sur une phase solide, dans lequel l'anticorps A et l'anticorps B sont étiquetés avec une matière d'étiquetage, et dans lequel l'insuline est mesurée par ELISA ou immunochromatographie.

11. Dosage d'insuline exogène comprenant les étapes de :

(1) obtention d'une concentration totale d'insuline humaine et d'insuline exogène;
(2) obtention d'une concentration d'insuline humaine avec le dosage d'insuline selon l'une quelconque des revendications 1 à 12; et
(3) obtention d'une concentration d'insuline exogène en soustrayant la concentration obtenue en (2) de la concentration obtenue en (1).

12. Anticorps anti-insuline humaine, dans lequel l'anticorps anti-insuline humaine est susceptible de reconnaître un épitope identique à un épitope reconnu par un anticorps monoclonal produit par l'hybridome du numéro matricule FERM BP-11314.

13. Réactif de dosage d'insuline, dans lequel le réactif de dosage d'insuline utilise l'anticorps selon la revendication 12.

14. Réactif de dosage d'insuline humaine utilisant les deux anticorps suivants :

1) l'anticorps anti-insuline humaine selon la revendication 12, et
2) un anticorps A ayant une propriété de réaction au moins avec de l'insuline humaine,

ou
utilisant les deux anticorps suivants :

1) l'anticorps anti-insuline humaine selon la revendication 12, et
2) un anticorps B ayant une propriété de reconnaissance spécifique de l'anticorps de 1).

15. Réactif de dosage d'insuline humaine selon la revendication 14, dans lequel les deux anticorps de 1) et 2) sont des anticorps monoclonaux,
ou
dans lequel l'anticorps de 1) est un anticorps monoclonal, et dans lequel l'anticorps de 2) est un anticorps polyclonal.

16. Réactif de dosage d'insuline humaine selon la revendication 14 ou 15, dans lequel l'anticorps de 1) et/ou l'anticorps de 2) sont immobilisés sur une phase solide.

**17.** Réactif de dosage d'insuline humaine selon la revendication 16, dans lequel la phase solide est du latex, et dans lequel l'insuline est dosée par un dosage d'immuno-agglutination de latex,
ou
dans lequel l'anticorps de 1) est immobilisé sur une phase solide, dans lequel l'anticorps de 2) est étiqueté avec une matière d'étiquetage, et dans lequel l'insuline est dosée par ELISA ou immunochromatographie.

**18.** Kit de dosage d'insuline exogène incluant les réactifs de dosage suivants :

(1) un réactif pour mesurer une concentration en insuline totale d'insuline humaine et d'insuline exogène, et
(2) le réactif de dosage d'insuline humaine selon l'une quelconque des revendications 13 à 17.

[FIGURE 1]

Insulin amino acid sequence

(f): Portion (f)
(g): Portion (g)

[FIGURE 2-1]

(a) 66224-antibody — Human insulin

(b) 66224-antibody — Proinsulin

(c) 66224-antibody — Insulin lispro

(d) 66224-antibody — Insulin aspart

[FIGURE 2-2]

(e) 66224-antibody — Insulin glargine

(f) 66224-antibody — Insulin detemir

(g) 66224-antibody — Insulin glulisine

(h) 66224-antibody — Porcine insulin

(i) 66224-antibody — Bovine insulin

[FIGURE 3-1]

( a ) 66408-antibody — Human insulin

( b ) 66408-antibody — Proinsulin

( c ) 66408-antibody — Insulin lispro

( d ) 66408-antibody — Insulin aspart

[FIGURE 3-2]

(e) 66408-antibody — Insulin glargine

(f) 66408-antibody — Insulin detemir

(g) 66408-antibody — Insulin glulisine

(h) 66408-antibody — Porcine insulin

(i) 66408-antibody — Bovine insulin

[FIGURE 4]

[FIGURE 5]

[FIGURE 6]

Primary antibody — Secondary antibody
66408-antibody — 66224-antibody

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H01148962 B
- JP H03118472 B
- JP S60188327 B
- JP 2010062261 W
- JP 2009295260 A

### Non-patent literature cited in the description

- *Clinical chemistry,* 2001, vol. 47 (3), 602-5
- *Clinical laboratory,* 2003, vol. 49 (3-4), 113-21
- *Clinical chemistry,* 2004, vol. 50 (1), 257-9
- Antibodies, A Laboratory Manual. Cold Spring Harbor Laboratory Press, 1988